(19) Europäisches Patentamt
European Patent Office
Office européen des brevets

(11) **EP 4 180 453 A1**

(12) **EUROPEAN PATENT APPLICATION**
published in accordance with Art. 153(4) EPC

(43) Date of publication:
**17.05.2023 Bulletin 2023/20**

(21) Application number: **21838564.9**

(22) Date of filing: **07.07.2021**

(51) International Patent Classification (IPC):
**C07K 14/705** (2006.01)   **C07K 16/22** (2006.01)
**A61K 38/00** (2006.01)   **A61P 27/02** (2006.01)

(52) Cooperative Patent Classification (CPC):
**A61K 38/00; A61P 27/02; C07K 14/705;
C07K 16/22**

(86) International application number:
**PCT/KR2021/008681**

(87) International publication number:
**WO 2022/010271 (13.01.2022 Gazette 2022/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **07.07.2020  KR 20200083536**

(71) Applicant: **Kanaph Therapeutics Inc.
Seoul 04348 (KR)**

(72) Inventors:
• **CHUNG, Eu Ddeum
Seongnam-si Gyeonggi-do 13476 (KR)**

• **RYU, Soomin
Anyang-si Gyeonggi-do 14109 (KR)**
• **KIM, Donggeon
Seoul 05629 (KR)**
• **CHANG, Jihoon
Seoul 04211 (KR)**
• **LEE, Byoung Chul
Seoul 06289 (KR)**

(74) Representative: **HGF
HGF Limited
1 City Walk
Leeds LS11 9DX (GB)**

Remarks:
The complete document including Reference
Table(s) and the Sequence Listing(s) can be
downloaded from the EPO website

(54) **FUSION PROTEIN INCLUDING COMPLEMENT PATHWAY INHIBITOR AND ANGIOGENESIS INHIBITOR AND USE THEREOF**

(57)    Provided is a fusion protein dimer containing an extracellular domain of CRIg or a fragment thereof, and a protein that specifically binds to VEGF. The protein may not only inhibit complement-related pathways, but also effectively modulate angiogenesis. Therefore, the fusion protein dimer may be effectively used for the treatment and prevention of complement-related diseases, specifically, eye diseases such as macular degeneration, and thus a high possibility of being industrially used.

【Fig. 5】

**Description**

**BACKGROUND**

The present invention relates to a fusion protein containing a complement pathway-inhibiting protein and an angiogenesis-inhibiting protein; and a composition for treating an eye disease, specifically, macular degeneration using the same.

The macular degeneration refers to a disease characterized by loss of central vision associated with abnormalities of Bruch's membrane, choroid, neural retina, and/or retinal pigment epithelium. A macula having a diameter of about 1/3 to 1/2 cm is present at the center of the retina. Below the retina, the choroid, which is a collection of blood vessels embedded in fibrous tissue, and the pigment epithelium (PE), which is provided above the choroid layer, are present. In this case, choroidal blood vessels provide nutrients to the retina. The choroid and PE are found in the front part of the eye.

Age-related macular degeneration (AMD), which is one type of the macular degeneration, is a disease associated with progressive loss of vision at the central part of the visual field, changes in color discrimination, and abnormal dark adaptation and sensitivity. AMD is roughly categorized as dry or wet AMD. Dry AMD is associated with the atrophic cell death of the central retina or macula required for fine vision used for activities such as reading, driving, or facial recognition. About 10% to 20% of patients with the dry AMD progress to type 2 AMD known as wet AMD.

The most significant risk factors in the two forms of the onset of the disease are age, and deposition of drusen, which are abnormal extracellular deposits, behind the retinal pigment epithelium. The drusen are characteristic deposits associated with AMD. The drusen are known to include a complement activator, an inhibitor, an activation-specific complement fragment, and a terminal pathway factor such as a cell membrane attack complex (MAC or C5b-9). In addition, wet AMD is associated with choroidal neovascularization (CNV). The pathogenic mechanism of the new choroidal neovascularization is little known, but factors such as inflammation, ischemia, and local production of angiogenic factors are considered to be important.

Meanwhile, a complement system is a critical component of innate immunity to microbial infection, and includes a population of proteins which are normally present in an inactive state in serum. The proteins are activated through the classical pathway, the lectin pathway, and the alternative pathway. Molecules on the surfaces of microorganisms activate the aforementioned pathways to cause the formation of a protease complex known as a C3-convertase.

Activation of the complement pathways produces biologically active fragments, including of complement proteins, for example, anaphylatoxins such as C3a and C5a and a C5b-9 cell membrane attack complex (MAC), which mediate the inflammatory response in leukocyte chemotaxis, activation of macrophages, neutrophils, platelets, mast cells, and endothelial cells, increased vascular permeability, cell lysis, and tissue damage. Moreover, it has been reported that some of the eye diseases are complement-related (Japanese Patent Application No. 2007-536964). However, the needs for drugs for treating effectively an eye disease, in particular, macular degeneration have been increasing so far, and thus studies on a therapeutic agent for macular degeneration are still in progress.

**SUMMARY**

Accordingly, as a result of studies to effectively treat and prevent an eye disease, in particular, macular degeneration, the present inventors have confirmed that a fusion protein, which blocks a complement-related pathway and an angiogenic pathway, may be used as a therapeutic agent for macular degeneration, thereby completing the present invention.

One aspect of the present invention provides a fusion protein containing: an extracellular domain of a complement receptor of the immunoglobulin superfamily (CRIg) or a fragment thereof; and a protein that specifically binds to a vascular endothelial growth factor (VEGF).

Another aspect of the present invention provides a fusion protein dimer in which the two fusion proteins are linked.

Yet another aspect of the present invention provides a polynucleotide encoding the fusion protein.

Still another aspect of the present invention provides a vector containing the polynucleotide.

Yet still another aspect of the present invention provides a transformed cell into which the vector is introduced.

Yet still another aspect of the present invention provides a pharmaceutical composition for treating or preventing an eye disease including, as an active ingredient, the fusion protein or the fusion protein dimer.

Yet still another aspect of the present invention provides a use of a fusion protein or a dimer thereof for treating an eye disease, wherein the fusion protein contains an extracellular domain of a CRIg or a fragment thereof, and a protein that specifically binds to a VEGF.

Yet still another aspect of the present invention provides a use of a fusion protein or a dimer thereof for the manufacture of a medicament for treatment or prevention of an eye disease, wherein the fusion protein contains an extracellular domain of a CRIg or a fragment thereof, and a protein that specifically binds to a VEGF.

Yet still another aspect of the present invention provides a method for treating and/or preventing an eye disease, the method including: administering a fusion protein or a dimer thereof to a subject, wherein the fusion protein contains an extracellular domain of a CRIg or a fragment thereof and a protein that specifically binds to a VEGF.

The fusion protein, which is a protein for inhibiting complement-related pathways and contains an extracellular domain of a CRIg or a fragment thereof and a protein that specifically binds to a VEGF, may not only efficiently inhibit complement-related mechanisms but also efficiently inhibit angiogenesis. Therefore, eye diseases caused by the complement system and eye diseases caused by angiogenesis may be effectively treated or prevented. Consequently, the fusion protein may be usefully used to effectively treat macular degeneration, in particular, both dry macular degeneration and wet macular degeneration.

## BRIEF DESCRIPTION OF THE DRAWINGS

Exemplary embodiments can be understood in more detail from the following description taken in conjunction with the accompanying drawings, in which:

FIG. 1 is images showing the SDS-PAGE results of C1.01, C1.02, C1.03, C1.04, C1.05, and C1.06;

FIG. 2 is images showing the SDS-PAGE results of C1.03m, C1.04m, and aflibercept;

FIG. 3 is images showing the SDS-PAGE results of C1.01m, C1.02m, C1.06m, and C1.07m;

FIG. 4 is images showing the SDS-PAGE results of rabbit C3, rabbit C3b, rat C3, and rat C3b;

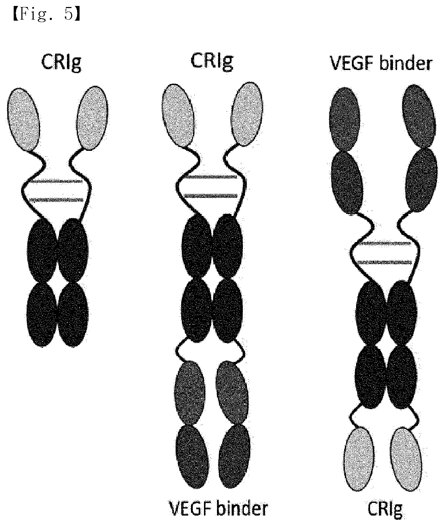

FIG. 5 is schematic diagrams of fusion proteins, and sequentially indicates CRIg-Fc (C1.01, left), CRIg-Fc-VEGF binder (C1.02, middle), and VEGF binder-Fc-CRIg (C1.04, right);

FIGS. 6a and 6b are graphs showing, by concentration, the binding affinity of C1.01 and C1.02 to human C3b and human VEGF165 through Biacore analysis;

FIGS. 7a and 7b are graphs showing, by concentration, the binding affinity of C1.01m and C1.02m to mouse C3b and human VEGF165 through Biacore analysis;

FIG. 8a is a graph showing the binding affinity of C1.01, C1.02, C1.03, and C1.04 to human C3b through ELISA;

FIG. 8b is a graph showing the binding affinity of aflibercept, C1.02, C1.04, and C1.05 to human VEGF165 through ELISA;

FIG. 8c is a graph showing the binding affinity of C1.01, C1.02, and C1.03 to mouse C3b through ELISA;

FIG. 8d is a graph showing the binding affinity of C1.02 to human C3b, human C2, and human C4 through ELISA;

FIG. 9a is a graph showing the hydrodynamic radius of C1.01 through dynamic light scattering analysis;

FIG. 9b is a graph showing the hydrodynamic radius of C1.02 through dynamic light scattering analysis;

FIG. 10 is a graph showing the viscosity according to the concentration of C1.02;

FIG. 11a is a graph showing the alternative complement pathway inhibiting effect of C1.01, C1.02, and C1.06 through hemolysis analysis (AH50);

FIG. 11b is a graph showing the alternative complement pathway inhibiting effect of C1.02, C1.04, C1.04m, and C1.05 through hemolysis analysis (AH50);

FIG. 11c is a graph showing the alternative complement pathway inhibiting effect of C1.01m, C1.02m, C1.06m, and C1.07m through hemolysis analysis (AH50);

FIG. 12a is a graph showing the classical complement pathway inhibiting effect of C1.01, C1.02, and C1.06 through hemolysis analysis (CH50);

FIG. 12b is a graph showing the classical complement pathway inhibiting effect of C1.02, C1.04, C1.04m, and C1.05 through hemolysis analysis (CH50);

FIG. 12c is a graph showing the classical complement pathway inhibiting effect of C1.01m, C1.02m, C1.06m, and C1.07m through hemolysis analysis (CH50);

FIG. 13 is a graph showing the VEGF signaling inhibiting effect of aflibercept, C1.01, C1.02, C1.05, and C1.06 using reporter cells;

FIG. 14 is a graph showing the VEGF signaling pathway inhibiting effect of C1.01, C1.02, and C1.05 through a wound healing assay method;

FIG. 15a is a graph showing the binding ability of C3b of a human and a cynomolgus monkey to C1.02, and the binding ability of C3b of a human and a cynomolgus monkey to C1.06 through ELISA;

FIG. 15b is a graph showing the binding ability of C3b of a human, a rat, and a rabbit to C1.02, and the binding ability of C3b of a human, a rat, and a rabbit to C1.06 through ELISA;

FIG. 15c is a graph showing the binding ability of the VEGFs of a human, a cynomolgus monkey, a rat, and a rabbit to C1.02 and the binding ability of the VEGFs of a human, a cynomolgus monkey, a rat, and a rabbit to C1.01 through ELISA;

FIG. 16a is images obtained by confirming, through fluorescein angiography, the choroidal neovascularization of each experimental group immediately after induction (Day 0) of mouse models for choroidal neovascularization to 7 days after the induction (Day 7);

FIG. 16b is a graph showing the body weight of each experimental group immediately after induction (Day 0) of the mouse models for choroidal neovascularization, 3 days after the induction (Day 3), and 7 days after the induction (Day 7);

FIG. 16c is a graph showing the quantification of the degree of choroidal neovascularization for each experimental group immediately after induction (Day 0) of the mouse models for choroidal neovascularization to 7 days after the induction (Day 7);

FIG. 17 is a graph showing the quantification of the choroidal neovascularization intensity for each experimental group 7 days and 14 days after induction of rabbit models for choroidal neovascularization;

FIG. 18a shows the quantification of the presence or absence of choroidal neovascularization in each experimental group immediately after induction of rat models for choroidal neovascularization and 10 days after the induction;

FIG. 18b shows the quantification of the vascular leakage area in each experimental group immediately after induction of the rat models for choroidal neovascularization and 10 days after the induction;

FIG. 19 is a view showing images showing an outer nuclear layer (ONL) of each experimental group of models for dry macular degeneration (FIGS. 19a to 19c), the cell count in the outer nuclear layer (FIG. 19d), the area of the outer nuclear layer (FIG. 19e), and the C3 expression level in the retina (FIG. 19f);

FIG. 20a is a graph showing the concentration of C1.02 in the vitreous humor after administration of 2,500 μg of C1.02 to rabbits via an intravitreal injection; and

FIG. 20b is a graph showing the concentration of C1.02 in the aqueous humor after administration of 2,500 μg of C1.02 to rabbits via an intravitreal injection.

## DETAILED DESCRIPTION OF EMBODIMENTS

### Fusion protein containing extracellular domain of CRIg

One aspect of the present invention provides a fusion protein containing: an extracellular domain of a complement receptor of the immunoglobulin superfamily (CRIg) or a fragment thereof; and a protein that specifically binds to a vascular endothelial growth factor (VEGF).

The term "CRIg" used in the present specification refers to an complement receptor immunoglobulin encoded by a VSIG4 gene, and also called protein Z39Ig. The CRIg is a receptor belonging to the type 4 complement receptor among the four types of complement receptors, and is expressed on the surfaces of macrophages such as Kupffer cells that perform phagocytosis in the liver. The CRIg is a membrane protein (integral membrane protein) bound to an extracellular region including an immunoglobulin domain. The CRIg binds to complement fragments C3b and iC3b, and functions to recognize and eliminate, by phagocytes, bacteria entering the human body or infectious bacteria in the blood.

The CRIg includes an isoform or spliced form of the CRIg. The isoform includes CRIg isoform 1, 2, or 3. The spliced form includes CRIg(L) or CRIg(S). The CRIg(L) may include V and C2-type terminal Ig domains, and the CRIg(S) may include only a V-type domain. Specifically, the CRIg(S) may contain the sequence of SEQ ID NO: 20.

The extracellular domain of the CRIg may be a portion of the receptor excluding transmembrane domain and cytoplasmic domain portions.

The CRIg may include a fragment of the extracellular domain of the CRIg. The fragment of the extracellular domain of the CRIg refers to a cleaved form having an activity equivalent to or similar to that of the extracellular domain of the CRIg. Specifically, the fragment refers to a fragment of a CRIg having an activity of promoting a complement action or phagocytosis by binding to C3b or iC3b.

In one embodiment, the CRIg may contain the amino acid sequence of SEQ ID NO: 19, SEQ ID NO: 20, SEQ ID NO: 21, SEQ ID NO: 22, SEQ ID NO: 74, or SEQ ID NO: 151. Specifically, a human CRIg may contain the amino acid sequence of SEQ ID NO: 19, SEQ ID NO: 20, or SEQ ID NO: 151, and a mouse CRIg may contain the amino acid sequence of SEQ ID NO: 21. Moreover, an extracellular domain of the mouse CRIg may contain the amino acid sequence of SEQ ID NO: 75, and an extracellular domain of the human CRIg may contain the amino acid sequence of SEQ ID NO: 22 or SEQ ID NO: 74.

The term "vascular endothelial growth factor (VEGF)" used in the present specification refers to a vascular endothelial growth factor, which is produced by cells that stimulate angiogenesis. The VEGF is an important signaling protein involved in angiogenesis by dividing and proliferating vascular endothelial cells and increasing vascular permeability. The VEGF is known to stimulate abnormal vascular proliferation in the retina in wet AMD.

Furthermore, in the present specification, VEGF or a VEGF-based protein may be collectively referred to as the term "VEGF". The VEGF-based protein may have an activity equivalent to or similar to that of the VEGF. Here, the "activity" may refer to, for example, specific binding to a VEGF receptor, and this specific binding may be measured by a method known to those skilled in the art.

In one embodiment, the VEGF-based protein may be one or more selected from the group consisting of VEGF-A, VEGF-B, VEGF-C, VEGF-D, VEGF-E, a placental growth factor (PlGF), and a recombinant VEGF. Preferably, the VEGF may be VEGF-A or B, or a PlGF.

The term "placental growth factor (PlGF)" used in the present specification refers to a transmembrane protein encoded by chromosome 2p21-p16. The PlGF acts as a selective ligand for VEGFR-1 and may promote angiogenesis. The PlGF has an identity of 40% or greater with the amino acid composition of a VEGF. In one embodiment, the PlGF may be PlGF-1 or PlGF-2.

The term "recombinant VEGF" used in the present specification refers to a VEGF recombined through alternative exon splicing. The recombinant VEGF may be VEGF111, VEGF121, VEGF145, VEGF148, VEGF165, VEGF183, VEGF189, or VEGF206 according to the number of amino acids. In one embodiment, the recombinant VEGF may be VEGF165.

The term "protein that specifically binds to VEGF" used in the present specification refers to an antibody that specifically binds to VEGF, or an extracellular domain of VEGF receptor.

The term "VEGF receptor" used in the present specification refers to a receptor that binds to VEGF. In this case, the VEGF receptor may be any one selected from the group consisting of VEGF receptor 1 (VEGFR-1), VEGF receptor 2 (VEGFR-2), and VEGF receptor 3 (VEGFR-3). The VEGF receptors include an immunoglobulin (Ig)-like domain, an extracellular domain, and an intracellularly separated kinase domain. The VEGF receptor is activated by binding to a VEGF ligand, so that dimerization or phosphorylation may occur. In one embodiment, the VEGF receptor may be VEGF receptor 1 or 2.

The term "extracellular domain of the VEGF receptor" used in the present specification refers to a domain of the VEGF receptor that binds to the VEGF. Specifically, the extracellular domain of the VEGF receptor refers to an extracellular domain portion including a ligand of VEGF, except for the transmembrane region and the cytoplasmic region of the VEGF receptor.

Moreover, the extracellular domain of the VEGF receptor may be a fragment of a VEGF receptor that binds to VEGF. In one embodiment, the fragment of the VEGF receptor includes a domain D1, D2, or D3 of the VEGF receptor, which is a region to which VEGF binds, or a combination thereof. Preferably, the fragment of the VEGF receptor may include D2 and D3.

In one embodiment, the extracellular domain of the VEGF receptor includes the D2 and D3 domains of VEGF receptor 1 or 2. Moreover, the extracellular domain of the VEGF receptor may bind to VEGF-A, VEGF-B, or PlGF to inhibit angiogenesis. In this case, one embodiment of the extracellular domain of the VEGF receptor may contain the amino acid sequence of SEQ ID NO: 14. Furthermore, the extracellular domain of the VEGF receptor may be in a form in which a portion of the extracellular domain of the VEGF receptor containing SEQ ID NO: 14 is cleaved or altered.

The term "antibody that specifically binds to VEGF" used in the present specification refers to an antibody, which causes an antigen-antibody reaction by specifically binding to VEGF, or a fragment thereof, and also called an anti-VEGF antibody.

The antibody is a generic term for molecules capable of forming specific antigen-antibody binding with VEGF. Moreover, the antibody may be used in any form as long as the antibody contains an antigen-binding domain capable of specifically binding to VEGF. The antibody or a fragment thereof may be a fragment antigen binding (Fab), an F(ab)$_2$, a single-chain variable fragment (scFv), a di-scFv, a single domain antibody (sdAb), a chimeric antibody, a humanized antibody, a human antibody, or a variant thereof.

The anti-VEGF antibody may be a nanobody. In this case, the antibody may contain CDR1 of SEQ ID NO: 49, CDR2 of SEQ ID NO: 50, and CDR3 of SEQ ID NO: 51. Specifically, the antibody may be BI-836880.

The anti-VEGF antibody may include a variable region of any one selected from the group consisting of aflibercept, bevacizumab, ranibizumab, ramucirumab, brolucizumab, faricimab, KSI-301, vanucizumab, BI-836880, HuMab G6-31, B20-4.1, BAT-5906, navicixizumab, dilpacimab, hPV-19, and AT-001. Preferably, the anti-VEGF antibody may include a variable region of aflibercept, bevacizumab, ranibizumab, brolucizumab, KSI-301, vanucizumab, BI-836880, or BAT-5906.

In this case, the aflibercept refers to a recombinant humanized fusion protein that inhibits VEGF-A and PlGF in blood vessels. Here, the aflibercept may be directly injected into the eyeball. The bevacizumab is an antibody which is an angiogenesis inhibitor that inhibits VEGF-A in blood vessels to inhibit the growth of the blood vessels. In the case of the treatment of macular degeneration, the bevacizumab may be directly injected into the eyeball. The ranibizumab is a Fab having the effect of treating wet macular degeneration by inhibiting angiogenesis. The ramucirumab is an angiogenesis-mediated substance, or an antibody that inhibits VEGF receptor 2. The brolucizumab is a scFv that binds to VEGF-A, inhibits angiogenesis, and treats wet macular degeneration. The faricimab is a bispecific antibody that inhibits VEGF-A and angiopoietin-2.

The KSI-301 is an antibody having the effect of treating wet macular degeneration. The vanucizumab is a bispecific humanized monoclonal antibody that inhibits VEGF-A and angiopoietin-2. The BI-836880 is a humanized bispecific nanobody that inhibits VEGF and angiopoietin-2. The G6-31 is a Fab fragment that inhibits human VEGF. The B20-4.1 is a scFv fragment that inhibits human VEGF.

The BAT-5906 is an antibody having the effect of treating wet macular degeneration. The navicixizumab is an anti-DLL4/VEGF bispecific antibody. The dilpacimab is an anti-DLL4/VEGF bispecific antibody and is also referred to as ABT-165. The hPV-19 is an antibody which is against VEGF and has an anti-angiogenic and anti-tumor activity. The AT-001 is an antibody that inhibits human VEGF receptor 3 to inhibit angiogenesis.

In one embodiment, the anti-VEGF antibody may include the variable region of BI-836880. Specifically, the antibody may include a heavy chain variable region containing CDR1 of SEQ ID NO: 49, CDR2 of SEQ ID NO: 50, and CDR3 of SEQ ID NO: 51.

In one embodiment, the anti-VEGF antibody may include the variable region of bevacizumab. Specifically, the antibody may include a heavy chain variable region containing HCDR1 of SEQ ID NO: 77, HCDR2 of SEQ ID NO: 78, and HCDR3 of SEQ ID NO: 79, and a light chain variable region containing LCDR1 of SEQ ID NO: 80, LCDR2 of SEQ ID NO: 81, and LCDR3 of SEQ ID NO: 82.

In one embodiment, the anti-VEGF antibody may include the variable region of ranibizumab. Specifically, the antibody may include a heavy chain variable region containing HCDR1 of SEQ ID NO: 83, HCDR2 of SEQ ID NO: 84, and HCDR3 of SEQ ID NO: 85, and a light chain variable region containing LCDR1 of SEQ ID NO: 86, LCDR2 of SEQ ID NO: 87, and LCDR3 of SEQ ID NO: 88.

In one embodiment, the anti-VEGF antibody may include the variable region of ramucirumab. Specifically, the antibody may include a heavy chain variable region containing HCDR1 of SEQ ID NO: 89, HCDR2 of SEQ ID NO: 90, and HCDR3 of SEQ ID NO: 91, and a light chain variable region containing LCDR1 of SEQ ID NO: 92, LCDR2 of SEQ ID NO: 93, and LCDR3 of SEQ ID NO: 94.

In one embodiment, the anti-VEGF antibody may include the variable region of faricimab. Specifically, the antibody may include a heavy chain variable region containing HCDR1 of SEQ ID NO: 101, HCDR2 of SEQ ID NO: 102, and HCDR3 of SEQ ID NO: 103, and a light chain variable region containing LCDR1 of SEQ ID NO: 104, LCDR2 of SEQ ID NO: 105, and LCDR3 of SEQ ID NO: 106.

In one embodiment, the anti-VEGF antibody may include the variable region of KSI-301. Specifically, the antibody may include a heavy chain variable region containing HCDR1 of SEQ ID NO: 107, HCDR2 of SEQ ID NO: 108, and HCDR3 of SEQ ID NO: 109, and a light chain variable region containing LCDR1 of SEQ ID NO: 110, LCDR2 of SEQ ID NO: 111, and LCDR3 of SEQ ID NO: 112.

In one embodiment, the anti-VEGF antibody may include the variable region of vanucizumab. Specifically, the antibody may include a heavy chain variable region containing HCDR1 of SEQ ID NO: 113, HCDR2 of SEQ ID NO: 114, and HCDR3 of SEQ ID NO: 115, and a light chain variable region containing LCDR1 of SEQ ID NO: 116, LCDR2 of SEQ ID NO: 117, and LCDR3 of SEQ ID NO: 118.

In one embodiment, the anti-VEGF antibody may include the variable region of BAT-5906. Specifically, the antibody may include a heavy chain variable region containing HCDR1 of SEQ ID NO: 119, HCDR2 of SEQ ID NO: 120, and HCDR3 of SEQ ID NO: 121, and a light chain variable region containing LCDR1 of SEQ ID NO: 122, LCDR2 of SEQ ID NO: 123, and LCDR3 of SEQ ID NO: 124.

In one embodiment, the anti-VEGF antibody may include the variable region of navicixizumab. Specifically, the antibody may include a heavy chain variable region containing HCDR1 of SEQ ID NO: 125, HCDR2 of SEQ ID NO: 126, and HCDR3 of SEQ ID NO: 127, and a light chain variable region containing LCDR1 of SEQ ID NO: 128, LCDR2 of SEQ ID NO: 129, and LCDR3 of SEQ ID NO: 130.

In one embodiment, the anti-VEGF antibody may include the variable region of dilpacimab. Specifically, the antibody may include a heavy chain variable region containing HCDR1 of SEQ ID NO: 131, HCDR2 of SEQ ID NO: 132, and HCDR3 of SEQ ID NO: 133, and a light chain variable region containing LCDR1 of SEQ ID NO: 134, LCDR2 of SEQ ID NO: 135, and LCDR3 of SEQ ID NO: 136.

In one embodiment, the anti-VEGF antibody may include the variable region of hPV-19. Specifically, the antibody may include a heavy chain variable region containing HCDR1 of SEQ ID NO: 137, HCDR2 of SEQ ID NO: 138, and HCDR3 of SEQ ID NO: 139, and a light chain variable region containing LCDR1 of SEQ ID NO: 140, LCDR2 of SEQ ID NO: 141, and LCDR3 of SEQ ID NO: 142.

In one embodiment, the anti-VEGF antibody may include the variable region of AT-001. Specifically, the antibody may include a heavy chain variable region containing HCDR1 of SEQ ID NO: 143, HCDR2 of SEQ ID NO: 144, and HCDR3 of SEQ ID NO: 145, and a light chain variable region containing LCDR1 of SEQ ID NO: 146, LCDR2 of SEQ ID NO: 147, and LCDR3 of SEQ ID NO: 148.

In one embodiment, the anti-VEGF antibody may include: a heavy chain of SEQ ID NO: 36 and a light chain of SEQ ID NO: 37; a heavy chain of SEQ ID NO: 38 and a light chain of SEQ ID NO: 39; a heavy chain of SEQ ID NO: 40 and a light chain of SEQ ID NO: 41; a heavy chain of SEQ ID NO: 43 and a light chain of SEQ ID NO: 44; a heavy chain of SEQ ID NO: 45 and a light chain of SEQ ID NO: 46; a heavy chain of SEQ ID NO: 47 and a light chain of SEQ ID NO: 48; a heavy chain of SEQ ID NO: 64 and a light chain of SEQ ID NO: 65; a heavy chain of SEQ ID NO: 66 and a light chain of SEQ ID NO: 67; a heavy chain of SEQ ID NO: 68 and a light chain of SEQ ID NO: 69; a heavy chain variable region of SEQ ID NO: 70 and a light chain variable region of SEQ ID NO: 71; or a heavy chain variable region of SEQ ID NO: 72 and a light chain variable region of SEQ ID NO: 73.

The fragment of the anti-VEGF antibody may be a single chain variable fragment (scFv). In this case, the scFv refers to a form in which a heavy chain variable region and a light chain variable region are linked by a peptide linker. Specifically, the scFv may include a variable region containing CDR1 of SEQ ID NO: 95, CDR2 of SEQ ID NO: 96, CDR3 of SEQ ID NO: 97, CDR4 of SEQ ID NO: 98, CDR5 of SEQ ID NO: 99, and LCDR6 of SEQ ID NO: 100. Moreover, the scFv may contain the amino acid sequence of SEQ ID NO: 42. In this case, one embodiment of the scFv may be brolucizumab.

The anti-VEGF antibody may include the variable region of HuMab G6-31 or B20-4.1. Specifically, the antibody may include a heavy chain variable region containing HCDR1 of SEQ ID NO: 52, HCDR2 of SEQ ID NO: 53, and HCDR3 of SEQ ID NO: 54, and a light chain variable region containing LCDR1 of SEQ ID NO: 55, LCDR2 of SEQ ID NO: 56, and LCDR3 of SEQ ID NO: 57. Moreover, the antibody may include a heavy chain variable region containing HCDR1 of SEQ ID NO: 58, HCDR2 of SEQ ID NO: 59, and HCDR3 of SEQ ID NO: 60, and a light chain variable region containing LCDR1 of SEQ ID NO: 61, LCDR2 of SEQ ID NO: 62, and LCDR3 of SEQ ID NO: 63.

The antibody that specifically binds to VEGF may refer to, without limitation, an antibody known to those skilled in the art. In another embodiment, the anti-VEGF antibody or a fragment thereof, which is disclosed in US Patent 9,527,925 B2, US Patent 8,268,314 B2, or US Publication 2019-0167790 A1, may be used as the antibody.

The extracellular domain of the CRIg or a fragment thereof, and the protein that specifically binds to VEGF may be linked by a linker.

Moreover, the extracellular domain of the CRIg or a fragment thereof, and an immunoglobulin fragment may be linked by a linker. The linker links two proteins. One embodiment of the linker may include 1 to 50 amino acids, albumin or a fragment thereof, or an Fc domain of an immunoglobulin. In this case, the Fc domain of the immunoglobulin refers to a protein which includes heavy chain constant region 2 (CH2) and heavy chain constant region 3 (CH3) of the immunoglobulin, but does not include variable regions of heavy and light chains and light chain constant region 1 (CH1) of the immunoglobulin. The immunoglobulin may be IgG, IgA, IgE, IgD, or IgM, and preferably IgG1. The Fc domain in the present specification may refer to a region including CH2 and CH3 domains, except for a hinge region.

In addition, the fusion protein may consist Structural Formula (I) or (II).

$$\text{N'-X-[linker (1)]n-Fc domain-[linker (2)]m-Y-C'} \quad \text{(I)}$$

$$\text{N'-Y-[linker (1)]n-Fc domain-[linker (2)]m-X-C'} \quad \text{(II)}$$

In Structural Formulae (I) and (II),

N' is the N-terminus of the fusion protein,
C' is the C-terminus of the fusion protein,
X is the extracellular domain of the CRIg or a fragment thereof,
Y is the protein that specifically binds to VEGF,
the linker (1) and the linker (2) are peptide linkers, and
n and m are each independently 0 or 1.

The protein that specifically binds to VEGF, the extracellular domain of the CRIg or a fragment thereof, and the Fc domain are as described above. The Fc domain may include CH2 and CH3 regions of an Fc heavy chain of the immunoglobulin. Moreover, the Fc domain of the immunoglobulin may be an Fc domain variant as well as a wild-type Fc domain. Further, the term "Fc domain variant" used in the present specification refers to an Fc domain which may have a glycosylation pattern different from that of the wild-type Fc domain, or may be in the form of increased glycosylation compared to the wild-type Fc domain, decreased glycosylation compared to the wild-type Fc domain, or being deglycosylated. Furthermore, an aglycosylated Fc domain is also included. The Fc domain or a variant thereof may have an adjusted number of sialic acids, fucosylations, and glycosylations through culture conditions, or genetic manipulation of a host.

In addition, the glycosylation of the Fc domain of the immunoglobulin may be modified by a conventional method, such as a chemical method, an enzymatic method, and a genetic engineering method using microorganisms. Moreover, the Fc domain variant may be in the form in which the Fc regions of immunoglobulin IgG, IgA, IgE, IgD, or IgM are mixed. Further, the Fc domain variant may be in a form in which some amino acids of the Fc domain are substituted with other amino acids.

The term "Fc domain variant" used in the present specification refers to a variant formed by altering the glycosylation of the wild-type Fc domain, an Fc domain in which sequences between Fc domains are mixed, or a variant formed by deleting, altering, substituting, and/or adding some amino acids of the wild-type Fc domain. The variant formed by deleting, altering, substituting, and/or adding some amino acids of the wild-type Fc domain may be prepared by a method known to those skilled in the art. In one embodiment, the Fc domain variant may be formed by substituting and/or adding some amino acid sequences of the wild-type Fc domain.

The "amino acid" introduced by substitution and/or addition may be any one selected from the group consisting of lysine (K), alanine (A), arginine (R), asparagine (N), aspartic acid (D), cysteine (C), glutamine (Q), glutamic acid (E), glycine (G), histidine (H), isoleucine (I), leucine (L), methionine (M), phenyl alanine (F), proline (P), serine (S), threonine (T), tryptophan (W), tyrosine (Y), and valine (V).

The Fc domain variation may be to regulate the activity or function of an antibody. In one embodiment, the Fc domain variation may be to regulate an effector function or cytotoxic activities of an antibody.

In one embodiment, the Fc domain variant may include a DANG variation or an NG variation. Moreover, the Fc domain variant may be a variant in which the 265th sequence in an IgG1 Fc domain is substituted from D to A, a variant in which the 297th sequence is substituted from N to G, or a combination thereof.

In one embodiment, the Fc domain may have any one amino acid sequence selected from the group consisting of SEQ ID NO: 6, SEQ ID NO: 11, and SEQ ID NO: 76. The Fc domain may be encoded by the polynucleotide sequence of SEQ ID NO: 28 or SEQ ID NO: 33.

The peptide linker (1) may consist of 5 to 80 consecutive amino acids, 20 to 60 consecutive amino acids, 25 to 50 consecutive amino acids, or 30 to 40 amino acids. In one embodiment, the peptide linker (1) may consist of 30 amino acids. Moreover, the peptide linker (1) may contain at least one cysteine. Specifically, one, two, or three cysteines may be contained. Further, the peptide linker (1) may be derived from a hinge of an immunoglobulin. In one embodiment, the peptide linker (1) may be a peptide linker consisting of the amino acid sequence of SEQ ID NO: 15 or 17.

The peptide linker (2) may consist of 1 to 50 consecutive amino acids, 3 to 30 consecutive amino acids, or 5 to 15 amino acids. In one embodiment, the peptide linker (2) may be (G4S)n (here, n is an integer of 1 to 10). In this case, n in (G4S)n may be 1, 2, 3, 4, 5, 6, 7, 8, 9, or 10. In one embodiment, the peptide linker (2) may be a peptide linker consisting of the amino acid sequence of SEQ ID NO: 16 or 18.

Preferably, the fusion protein may consist of Structural Formula (I).

In one embodiment, the fusion protein may contain any one amino acid sequence selected from the group consisting of SEQ ID NO: 2, SEQ ID NO: 4, SEQ ID NO: 8, and SEQ ID NO: 10.

In another embodiment, the fusion protein contains a polypeptide which has, with any one amino acid sequence selected from the group consisting of SEQ ID NO: 2, SEQ ID NO: 4, SEQ ID NO: 8, and SEQ ID NO: 10, a sequence identity of 85%, 86%, 87%, 88%, 89%, 90%, 91%, 92%, 93%, 94%, 95%, 96%, 97%, 98%, 99%, or 100%. In this case, the identity may be determined, for example, through percent homology, and homology comparison software such as BlastN software provided from the National Center for Biotechnology Information (NCBI).

**Fusion protein dimer**

Another aspect of the present invention provides a dimer in which two fusion proteins, which contain an extracellular domain of CRIg or a fragment thereof and a protein that specifically binds to VEGF, are linked.

In this case, the linkage between the fusion proteins constituting the dimer may be formed by a disulfide bond through cysteine present in the linker, but is not limited thereto. The fusion proteins constituting the dimer may be the same, but may be different from each other. Preferably, the dimer may be a homodimer.

**Polynucleotide encoding fusion protein**

Yet another aspect of the present invention provides a polynucleotide encoding a fusion protein, which contains an extracellular domain of CRIg or a fragment thereof and a protein that specifically binds to VEGF.

In one embodiment, the polynucleotide may contain a nucleic acid sequence which has, with SEQ ID NO: 2, SEQ ID NO: 4, SEQ ID NO: 8, or SEQ ID NO: 10, an identity of at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100%.

In one embodiment, the polynucleotide may have, with any one base sequence selected from the group consisting of SEQ ID NO: 24, SEQ ID NO: 26, SEQ ID NO: 30, and SEQ ID NO: 32, an identity of at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100%.

The polynucleotide may further contain a nucleic acid encoding a signal sequence or a leader sequence. The term "signal sequence" used in the present specification refers to a signal peptide that directs the secretion of a target protein. The signal peptide is translated in a host cell and then cleaved. Specifically, the signal sequence is an amino acid sequence that initiates the transportation of a protein across an endoplasmic reticulum (ER) membrane.

Such characteristics of the signal sequence are well known in the art, and the signal sequence typically contains 16 to 30 amino acid residues, but may contain more or fewer amino acid residues than the aforementioned number of amino acid residues. A typical signal peptide consists of three regions: a basic N-terminal region; a central hydrophobic region; and a more polar C-terminal region. The central hydrophobic region contains 4 to 12 hydrophobic residues that fix the signal sequence through a membrane lipid bilayer during the transportation of an immature polypeptide.

After the initiation, the signal sequence is cleaved in the lumen of the ER by cell enzymes commonly known as signal peptidases. In this case, the signal sequence may be tissue plasminogen activation (tPa), a signal sequence of Herpes simplex virus glycoprotein D (HSV gDs), or a growth hormone secretion signal sequence. Preferably, a secretion signal sequence used in higher eukaryotic cells including mammals and the like may be used. Moreover, as the signal sequence, a wild-type signal sequence may be used, or a signal sequence substituted with a codon having a high expression frequency in a host cell may be used.

## Vector carrying polynucleotide encoding fusion protein

Still another aspect of the present invention provides a vector including the aforementioned polynucleotide.

The vector may be introduced into a host cell, and recombined and inserted into a host cell genome. Alternatively, the vector is understood to be a nucleic acid means containing a polynucleotide sequence capable of being spontaneously replicated as an episome. The vector includes a linear nucleic acid, a plasmid, a phagemid, a cosmid, an RNA vector, a viral vector, and analogs thereof. Examples of the viral vector include a retrovirus, an adenovirus, and an adeno-associated virus, but are not limited to these examples.

Specifically, the vector may be plasmid DNA, phage DNA, or the like, and may be a commercially developed plasmid (pUC18, pBAD, pIDTSAMRT-AMP, or the like), an *Escherichia* coli-derived plasmid (pYG601BR322, pBR325, pUC118, pUC119, or the like), a *Bacillus subtilis-derived* plasmid (pUB110, pTP5, or the like), a yeast-derived plasmid (YEp13, YEp24, YCp50, or the like), phage DNA (Charon4A, Charon21A, EMBL3, EMBL4, λgt10, kgt11, λZAP, or the like), an animal viral vector (a retrovirus, an adenovirus, a vaccinia virus, or the like), or an insect virus vector (a baculovirus or the like). Since the vector shows different protein expression levels and modifications depending on the host cell, it is preferable to select and use a host cell most suitable for the purpose.

The term "gene expression" or "expression" of the target protein used in the specification is understood to mean the transcription of a DNA sequence, the translation of an mRNA transcript, and the secretion of a fusion protein product or a fragment thereof. A useful expression vector may be RcCMV (Invitrogen, Carlsbad) or a variant thereof. The expression vector may include a human cytomegalovirus (CMV) promoter for promoting continuous transcription of target genes in mammalian cells, and a bovine growth hormone polyadenylation signal sequence for increasing the steady-state level of RNA after transcription.

## Transformed cell expressing fusion protein

Yet still another aspect of the present invention provides a transformed cell into which the aforementioned vector is introduced.

Examples of a host cell of the transformed cell may include a prokaryotic cell, a eukaryotic cell, and cells of mammals, plants, insects, fungi, or cellular origin, but are not limited to these examples. As an example of the prokaryotic cell, *Escherichia coli* may be used. Moreover, as an example of the eukaryotic cell, a yeast may be used. Furthermore, as the mammalian cells, CHO cells, F2N cells, CSO cells, BHK cells, Bowes melanoma cells, HeLa cells, 911 cells, AT1080 cells, A549 cells, HEK 293 cells, HEK293T cells, or the like may be used, but the present invention is not limited thereto. Any cells, which may be used as a mammalian host cell known to those skilled in the art, may all be used.

In addition, when introducing the expression vector into the host cell, a $CaCl_2$ precipitation method, a Hanahan method in which efficiency is increased by using a reducing substance called dimethyl sulfoxide (DMSO) in the $CaCl_2$ precipitation method, an electroporation, a calcium phosphate precipitation method, a plasmogamy method, a stirring method using silicon carbide fibers, an agrobacterium-mediated transformation method, a transformation method using PEG, dextran sulfate, lipofectamine, and a drying/inhibition-mediated transformation method, and the like may be used.

As described above, for optimizing the properties of the fusion protein as a therapeutic agent, or for other purposes, the glycosylation pattern (for example, sialic acid, fucosylation, and glycosylation) of the fusion protein may be adjusted by manipulating glycosylation-related genes of the host cell through a method known to those skilled in the art.

## Method for producing fusion protein

Yet still another aspect of the present invention provides a method for producing a fusion protein or a dimer thereof, the method including: culturing the transformed cell, wherein the fusion protein contains an extracellular domain of a CRIg or a fragment thereof and a protein that specifically binds to a VEGF.

The production method may include: i) culturing the transformed cell to obtain cultured medium; and ii) collecting a fusion protein or a dimer thereof from the cultured medium.

The method for culturing the transformed cell may be carried out using a method widely known in the art. Specifically, the culturing may be continuously performed in a batch process, or a fed batch or repeated fed batch process.

## Use of fusion protein or dimer thereof

Yet still another aspect of the present invention provides a pharmaceutical composition for treating or preventing an eye disease, including, as an active ingredient, the fusion protein, or a fusion protein dimer in which the two fusion proteins are bound.

The fusion protein and the fusion protein dimer are as described above.

The term "eye disease" used in the present specification may be a generic term for diseases in which the eye is the site of a disease. The eye disease may refer to an eye disease triggered or aggravated by a complement activity or angiogenesis, or an eye disease including excessive angiogenesis as a major disease symptom. The eye disease may be any one selected from the group consisting of age-related macular degeneration (AMD), geographic atrophy (GA), choroidal neovascularization (CNV), uveitis, diabetic and other ischemia-related retinopathy, diabetic macular edema, pathological myopia, a von Hippel-Lindau disease, ocular histoplasmosis, central retinal vein occlusion (CRVO), corneal angiogenesis, and retinal angiogenesis.

The extracellular domain of the CRIg or a fragment thereof, and the protein that specifically binds to VEGF are as described above.

The preferred dosage of the pharmaceutical composition varies depending on the conditions and body weight of a patient, the severity of a disease, a drug form, and the route and duration of administration, but may be appropriately selected by those skilled in the art. The active ingredient in the pharmaceutical composition for treating or preventing an eye disease according to the present invention may be contained in any amount (effective amount) according to a use, a dosage form, a blending purpose, or the like as long as the active ingredient exhibits an activity of treating an eye disease, or, in particular, may exhibit a therapeutic effect on macular degeneration, but a typical effective amount would be determined within the range of 0.001 wt% to 20.0 wt%, based on the total weight of the composition. The term "effective amount" used in the present specification refers to an amount of an active ingredient capable of inducing the effect of ameliorating or treating the state of an eye disease, in particular, the effect of ameliorating or treating the state of macular degeneration. Such an effective amount may be experimentally determined within the ordinary ability of those skilled in the art.

The term "treatment" used in the present specification may be used in the sense that the term includes both a therapeutic treatment and a preventive treatment. In this case, the prevention may be used to mean alleviating or reducing pathological conditions or a disease of a subject. In one embodiment, the term "treatment" includes any form of administration or application for treating a disease in mammals, including a human. Moreover, the term includes inhibiting or slowing a disease or the progression of the disease, and includes the meaning of: restoring or repairing a damaged or lost function to partially or completely alleviate a disease; stimulating inefficient processes; or alleviating a serious disease.

Pharmacokinetic parameters such as bioavailability and underlying parameters such as a clearance rate may also affect efficacy. Therefore, "enhanced efficacy" (for example, improvement in efficacy) may be attributed to improved pharmacokinetic parameters and improved efficacy, and may be measured by comparing parameters such as a clearance rate and treatment or amelioration of an eye disease in an experimental animal or a human subject.

The term "therapeutically effective amount" or "pharmaceutically effective amount" used in the present specification refers to an amount of a compound or composition effective for preventing or treating a target disease, and also refers to an amount which is sufficient to treat a disease with a reasonable benefit/risk ratio applicable to a medical treatment, and does not cause side effects. The level of the effective amount may be determined according to factors including health conditions of a patient, kinds of disease, severity, a drug activity, sensitivity to drugs, an administration method, an administration time, an administration route, an excretion rate, a treatment duration, a combination, or concurrently used drugs, and other factors well known in the medical field. In one embodiment, the therapeutically effective amount refers to an amount of a drug effective for treating an eye disease.

In this case, the pharmaceutical composition may further contain a pharmaceutically acceptable carrier. The pharmaceutically acceptable carrier may be any carrier as long as the carrier is a non-toxic substance suitable for delivery to a patient. Distilled water, alcohol, fat, wax, and inert solids may be contained as carriers. A pharmaceutically acceptable adjuvant (buffer or dispersant) may also be contained in the pharmaceutical composition.

Specifically, the pharmaceutical composition contains the pharmaceutically acceptable carrier in addition to the active ingredient, and may be prepared in a parenteral dosage form according to the administration route by a conventional method known in the art. Here, the expression "pharmaceutically acceptable" means that it does not inhibit the activity of the active ingredient, and does not have toxicity beyond what the application (prescription) target is adaptable.

When the pharmaceutical composition is prepared in a parenteral dosage form, the pharmaceutical composition may be formulated in the form of an injection, a transdermal administration agent, a nasal inhalant, or a suppository according to a method known in the art, together with a suitable carrier. When formulated as an injection, sterile water, ethanol, a polyol such as glycerol or propylene glycol, or a mixture thereof may be used as the suitable carrier, and preferably Ringer's solution, phosphate buffered saline (PBS) containing triethanolamine, an isotonic solution such as sterile water for injection or 5% dextrose, or the like may be used. The formulation of the pharmaceutical composition is known in the art, and specifically, reference may be made to the document [Remington's Pharmaceutical Sciences (19th ed., 1995)] or the like. The document is incorporated to be a part of the present specification.

The preferred dosage of the pharmaceutical composition may be in the range of 0.01 $\mu$g/kg to 10 g/kg or the range of 0.01 mg/kg to 1 g/kg per day, according to the conditions, body weight, gender, or age of a patient, the severity of a patient, or an administration route. The administration may be performed once a day or may be dividedly performed several times. Such a dosage should not be comprehended as limiting the scope of the present invention in any aspect.

Subjects to which the pharmaceutical composition may be applied (prescribed) are mammals and humans, and particularly preferably humans. The pharmaceutical composition of the present application may further contain, in addition to the active ingredient, any compound or natural extract known to have the effect of treating an eye disease, in particular, macular degeneration.

Yet still another aspect of the present invention provides a use of a fusion protein or a dimer thereof, which is for treating an eye disease, wherein the fusion protein contains an extracellular domain of CRIg or a fragment thereof and a protein that specifically binds to VEGF.

Yet still another aspect of the present invention provides a use of a fusion protein or a dimer thereof for the manufacture of a medicament for treatment or prevention of an eye disease, wherein the fusion protein contains an extracellular domain of CRIg or a fragment thereof and a protein that specifically binds to VEGF.

Yet still another aspect of the present invention provides a method for treating and/or preventing an eye disease, the method including: administering a fusion protein or a dimer thereof to a subject, wherein the fusion protein contains an extracellular domain of CRIg or a fragment thereof and a protein that specifically binds to VEGF.

The fusion protein, the dimer, and the eye disease are as described above. In this case, the subject may be a subject suffering from an eye disease. Moreover, the subject may be a mammal and preferably a human.

Regarding the administration route, dosage, and administration frequency of the fusion protein or fusion protein dimer, the administration to the subject may be performed in various manners and amounts according to the conditions of a patient and the presence or absence of side effects, and the optimal administration method, dosage, and administration frequency could be selected within an appropriate range by those skilled in the art. Moreover, the fusion protein or fusion protein dimer may be administered in combination with other drugs or physiologically active substances having known therapeutic effects for the disease to be treated, or may be formulated in the form of a combination formulation with other drugs.

In one embodiment, the fusion protein may inhibit a complement pathway, phagocytosis, and/or angiogenesis. Therefore, the fusion protein may be effectively used for an eye disease such as wet or dry macular degeneration. In particular, it was confirmed that the case of combining an extracellular domain of CRIg or a fragment thereof, and a protein that specifically binds to VEGF had a higher effect and also had a synergistic effect compared to the case of including one of them. Consequently, the fusion protein may effectively treat dry and wet macular degeneration by effectively inhibiting a complement pathway and angiogenesis.

Hereinafter, the present invention will be described in more detail with reference to the following Examples. Here, the following Examples are only for illustratively describing the present invention, and do not limit the scope of the present invention.

**Preparation Example 1. Preparation of fusion protein containing** CRIg

[Table 1]

| Code | Format | Description | Corresponding sequence |
|------|--------|-------------|------------------------|
| C1.01 | Fc-fusion | hu CRIg-hu IgG1 Fc DANG | SEQ ID NO: 1 |

(continued)

| Code | Format | Description | Corresponding sequence |
|------|--------|-------------|------------------------|
| C1.02 | Fc-fusion | hu CRIg-hu IgG1 Fc DANG-VEGF binder | SEQ ID NO: 2 |
| C1.03 | Fc-fusion | hu IgG1 Fc DANG-hu CRIg | SEQ ID NO: 3 |
| C1.04 | Fc-fusion | VEGF binder-hu IgG1 Fc DANG-hu CRIg | SEQ ID NO: 4 |
| C1.05 | Fc-fusion | VEGF binder-hu IgG1 Fc DANG | SEQ ID NO: 5 |
| C1.06 | Fc-fusion | hu IgG1 Fc DANG | SEQ ID NO: 6 |
| C1.01m | Fc-fusion | mu CRIg-mu IgG2a Fc DANG | SEQ ID NO: 7 |
| C1.02m | Fc-fusion | mu CRIg-mu IgG2a Fc DANG-VEGF binder | SEQ ID NO: 8 |
| C1.03m | Fc-fusion | mu IgG2a Fc DANG-mu CRIg | SEQ ID NO: 9 |
| C1.04m | Fc-fusion | VEGF binder-mu IgG2a Fc DANG-mu CRIg | SEQ ID NO: 10 |
| C1.06m | Fc-fusion | mu IgG2a Fc DANG | SEQ ID NO: 11 |
| C1.07m | Fc-fusion | mu IgG2a Fc DANG-VEGF binder | SEQ ID NO: 12 |
| Aflibercept | Fc-fusion | Aflibercept | SEQ ID NO: 13 |

C1.01 (SEQ ID NO: 1) consists of an extracellular domain region (20 to 283) of a human CRIg protein, a linker, and human IgG1 Fc in which the effector function is removed through the DANG mutation (D265A, N297G).

C1.02 (SEQ ID NO: 2) consists of an extracellular domain region (20 to 283) of a human CRIg protein, a linker, human IgG1 Fc DANG, a linker, and a VEGF binding region of aflibercept.

C1.03 (SEQ ID NO: 3) consists of human IgG1 Fc DANG, a linker, and an extracellular domain region (20 to 283) of a human CRIg protein.

C1.04 (SEQ ID NO: 4) consists of a VEGF binding region of aflibercept, a linker, human IgG1 Fc DANG, a linker, and an extracellular domain region (20 to 283) of a human CRIg protein.

C1.05 (SEQ ID NO: 5) consists of a VEGF binding region of aflibercept, a linker, and human IgG1 Fc DANG.

C1.06 (SEQ ID NO: 6) consists of human IgG1 Fc DANG.

C1.01m (SEQ ID NO: 7) consists of an extracellular domain region (20 to 187) of a mouse CRIg protein, a linker, and mouse IgG2a Fc in which the effector function is removed through the DANG mutation (D265A, N297G).

C1.02m (SEQ ID NO: 8) consists of an extracellular domain region (20 to 187) of a mouse CRIg protein, a linker, mouse IgG2a Fc DANG, a linker, and a VEGF binding region of aflibercept.

C1.03m (SEQ ID NO: 9) consists of mouse IgG2a Fc DANG, a linker, and an extracellular domain region (20 to 187) of a mouse CRIg protein.

C1.04m (SEQ ID NO: 10) consists of a VEGF binding region of aflibercept, a linker, mouse IgG2a Fc DANG, a linker, and an extracellular domain region (20 to 187) of a mouse CRIg protein.

C1.06m (SEQ ID NO: 11) consists of mouse IgG2a Fc DANG.

C1.07m (SEQ ID NO: 12) consists of mouse IgG2a Fc DANG, a linker, and a VEGF binding region of aflibercept.

**[SEQ ID NO: 1] hu CRIg-hu IgG1 Fc DANG**

RPILEVPESVTGPWKGDVNLPCTYDPLQGYTQVLVKWLVQRG

SDPVTIFLRDSSGDHIQQAKYQGRLHVSHKVPGDVSLQLSTLEMDDRSHYTC

EVTWQTPDGNQVVRDKITELRVQKLSVSKPTVTTGSYGFTVPQGMRISLQ

CQARGSPPISYIWYKQQTNNQEPIKVATLSTLLFKPAVIADSGSYFCTAKGQV

GSEQHSDIVKFVVKDSSKLLKTKTEAPTTMTYPLKATSTVKQSWDWTTDMD

GYLGETSAGPGKSLPGGGGSDKTHTCPPCPAPELLGGPSVFLFPPKPKDTLMI

SRTPEVTCVVVAVSHEDPEVKFNWYVDGVEVHNAKTKPREEQYGSTYRVV

SVLTVLHQDWLNGKEYKCKVSNKALPAPIEKTISKAKGQPREPQVYTLPPSR

DELTKNQVSLTCLVKGFYPSDIAVEWESNGQPENNYKTTPPVLDSDGSFFLY

SKLTVDKSRWQQGNVFSCSVMHEALHNHYTQKSLSLSPGK

**[SEQ ID NO: 2] hu CRIg-hu IgG1 Fc DANG-VEGF binder**

RPILEVPESVTGPWKGDVNLPCTYDPLQGYTQVLVKWLVQRG

SDPVTIFLRDSSGDHIQQAKYQGRLHVSHKVPGDVSLQLSTLEMDDRSHYTC

EVTWQTPDGNQVVRDKITELRVQKLSVSKPTVTTGSYGFTVPQGMRISLQ

CQARGSPPISYIWYKQQTNNQEPIKVATLSTLLFKPAVIADSGSYFCTAKGQV

GSEQHSDIVKFVVKDSSKLLKTKTEAPTTMTYPLKATSTVKQSWDWTTDMD

GYLGETSAGPGKSLPGGGGSDKTHTCPPCPAPELLGGPSVFLFPPKPKDTLMI

SRTPEVTCVVVAVSHEDPEVKFNWYVDGVEVHNAKTKPREEQYGSTYRVV

SVLTVLHQDWLNGKEYKCKVSNKALPAPIEKTISKAKGQPREPQVYTLPPSR

DELTKNQVSLTCLVKGFYPSDIAVEWESNGQPENNYKTTPPVLDSDGSFFLY

SKLTVDKSRWQQGNVFSCSVMHEALHNHYTQKSLSLSPGGGGGSGGGGSS

DTGRPFVEMYSEIPEIIHMTEGRELVIPCRVTSPNITVTLKKFPLDTLIPDGKRII

WDSRKGFIISNATYKEIGLLTCEATVNGHLYKTNYLTHRQTNTIIDVVLSPSH

GIELSVGEKLVLNCTARTELNVGIDFNWEYPSSKHQHKKLVNRDLKTQSGSE

MKKFLSTLTIDGVTRSDQGLYTCAASSGLMTKKNSTFVRVHEK

**[SEQ ID NO: 3] hu IgG1 Fc DANG-hu CRIg**

DKTHTCPPCPAPELLGGPSVFLFPPKPKDTLMISRTPEVTCVVV

AVSHEDPEVKFNWYVDGVEVHNAKTKPREEQYGSTYRVVSVLTVLHQDWL

NGKEYKCKVSNKALPAPIEKTISKAKGQPREPQVYTLPPSRDELTKNQVSLT

CLVKGFYPSDIAVEWESNGQPENNYKTTPPVLDSDGSFFLYSKLTVDKSRWQ

QGNVFSCSVMHEALHNHYTQKSLSLSPGGGGGSGGGGSRPILEVPESVTGP

WKGDVNLPCTYDPLQGYTQVLVKWLVQRGSDPVTIFLRDSSGDHIQQAKY

QGRLHVSHKVPGDVSLQLSTLEMDDRSHYTCEVTWQTPDGNQVVRDKITEL

RVQKLSVSKPTVTTGSYGFTVPQGMRISLQCQARGSPPISYIWYKQQTNNQ

EPIKVATLSTLLFKPAVIADSGSYFCTAKGQVGSEQHSDIVKFVVKDSSKLLK

TKTEAPTTMTYPLKATSTVKQSWDWTTDMDGYLGETSAGPGKSLPG

**[SEQ ID NO: 4] VEGF binder-hu IgG1 Fc DANG-hu CRIg**

SDTGRPFVEMYSEIPEIIHMTEGRELVIPCRVTSPNITVTLKKFP

LDTLIPDGKRIIWDSRKGFIISNATYKEIGLLTCEATVNGHLYKTNYLTHRQT

NTIIDVVLSPSHGIELSVGEKLVLNCTARTELNVGIDFNWEYPSSKHQHKKLV

NRDLKTQSGSEMKKFLSTLTIDGVTRSDQGLYTCAASSGLMTKKNSTFVRV

HEKGGGGSDKTHTCPPCPAPELLGGPSVFLFPPKPKDTLMISRTPEVTCVVVA

VSHEDPEVKFNWYVDGVEVHNAKTKPREEQYGSTYRVVSVLTVLHQDWLN

GKEYKCKVSNKALPAPIEKTISKAKGQPREPQVYTLPPSRDELTKNQVSLTCL

VKGFYPSDIAVEWESNGQPENNYKTTPPVLDSDGSFFLYSKLTVDKSRWQQ

GNVFSCSVMHEALHNHYTQKSLSLSPGGGGGSGGGGSRPILEVPESVTGPW

KGDVNLPCTYDPLQGYTQVLVKWLVQRGSDPVTIFLRDSSGDHIQQAKYQG

RLHVSHKVPGDVSLQLSTLEMDDRSHYTCEVTWQTPDGNQVVRDKITELRV

QKLSVSKPTVTTGSYGFTVPQGMRISLQCQARGSPPISYIWYKQQTNNQEPI

KVATLSTLLFKPAVIADSGSYFCTAKGQVGSEQHSDIVKFVVKDSSKLLKTK

TEAPTTMTYPLKATSTVKQSWDWTTDMDGYLGETSAGPGKSLPG

**[SEQ ID NO: 5] VEGF binder-hu IgG1 Fc DANG**

SDTGRPFVEMYSEIPEIIHMTEGRELVIPCRVTSPNITVTLKKFP

LDTLIPDGKRIIWDSRKGFIISNATYKEIGLLTCEATVNGHLYKTNYLTHRQT

NTIIDVVLSPSHGIELSVGEKLVLNCTARTELNVGIDFNWEYPSSKHQHKKLV

NRDLKTQSGSEMKKFLSTLTIDGVTRSDQGLYTCAASSGLMTKKNSTFVRV

HEKGGGGSDKTHTCPPCPAPELLGGPSVFLFPPKPKDTLMISRTPEVTCVVVA

VSHEDPEVKFNWYVDGVEVHNAKTKPREEQYGSTYRVVSVLTVLHQDWLN

GKEYKCKVSNKALPAPIEKTISKAKGQPREPQVYTLPPSRDELTKNQVSLTCL

VKGFYPSDIAVEWESNGQPENNYKTTPPVLDSDGSFFLYSKLTVDKSRWQQ

GNVFSCSVMHEALHNHYTQKSLSLSPG

**[SEQ ID NO: 6] hu IgG1 Fc DANG**

SVFLFPPKPKDTLMISRTPEVTCVVVAVSHEDPEVKFNWYVDG

VEVHNAKTKPREEQYGSTYRVVSVLTVLHQDWLNGKEYKCKVSNKALPAP

IEKTISKAKGQPREPQVYTLPPSRDELTKNQVSLTCLVKGFYPSDIAVEWESN

GQPENNYKTTPPVLDSDGSFFLYSKLTVDKSRWQQGNVFSCSVMHEALHNH

YTQKSLSLSPG

**[SEQ ID NO: 7] mu CRIg-mu IgG2a Fc DANG**

HPTLKTPESVTGTWKGDVKIQCIYDPLRGYRQVLVKWLVRHG

SDSVTIFLRDSTGDHIQQAKYRGRLKVSHKVPGDVSLQINTLQMDDRNHYTC

EVTWQTPDGNQVIRDKIIELRVRKYNPPRINTEAPTTLHSSLEATTIMSSTSDL

TTNGTGKLEETIAGSGRNLPGGGGSEPRGPTIKPCPPCKCPAPNLLGGPSVFIF

PPKIKDVLMISLSPIVTCVVVASEDDPDVQISWFVNNVEVHTAQTQTHRED

YGSTLRVVSALPIQHQDWMSGKEFKCKVNNKDLPAPIERTISKPKGSVRAPQ

VYVLPPPEEEMTKKQVTLTCMVTDFMPEDIYVEWTNNGKTELNYKNTEPVL

DSDGSYFMYSKLRVEKKNWVERNSYSCSVVHEGLHNHHTTKSFSRTPGK

**[SEQ ID NO: 8] mu CRIg-mu IgG2a Fc DANG-VEGF binder**

HPTLKTPESVTGTWKGDVKIQCIYDPLRGYRQVLVKWLVRHG
SDSVTIFLRDSTGDHIQQAKYRGRLKVSHKVPGDVSLQINTLQMDDRNHYTC
EVTWQTPDGNQVIRDKIIELRVRKYNPPRINTEAPTTLHSSLEATTIMSSTSDL
TTNGTGKLEETIAGSGRNLPGGGGSEPRGPTIKPCPPCKCPAPNLLGGPSVFIF
PPKIKDVLMISLSPIVTCVVVAVSEDDPDVQISWFVNNVEVHTAQTQTHRED
YGSTLRVVSALPIQHQDWMSGKEFKCKVNNKDLPAPIERTISKPKGSVRAPQ
VYVLPPPEEEMTKKQVTLTCMVTDFMPEDIYVEWTNNGKTELNYKNTEPVL
DSDGSYFMYSKLRVEKKNWVERNSYSCSVVHEGLHNHHTTKSFSRTPGGG
GGSGGGGSSDTGRPFVEMYSEIPEIIHMTEGRELVIPCRVTSPNITVTLKKFPL
DTLIPDGKRIIWDSRKGFIISNATYKEIGLLTCEATVNGHLYKTNYLTHRQTN
TIIDVVLSPSHGIELSVGEKLVLNCTARTELNVGIDFNWEYPSSKHQHKKLVN
RDLKTQSGSEMKKFLSTLTIDGVTRSDQGLYTCAASSGLMTKKNSTFVRVHE
K

[SEQ ID NO: 9] mu IgG2a Fc DANG-mu CRIg

EPRGPTIKPCPPCKCPAPNLLGGPSVFIFPPKIKDVLMISLSPIVT
CVVVAVSEDDPDVQISWFVNNVEVHTAQTQTHREDYGSTLRVVSALPIQHQ
DWMSGKEFKCKVNNKDLPAPIERTISKPKGSVRAPQVYVLPPPEEEMTKKQ
VTLTCMVTDFMPEDIYVEWTNNGKTELNYKNTEPVLDSDGSYFMYSKLRVE
KKNWVERNSYSCSVVHEGLHNHHTTKSFSRTPGGGGGSGGGGSHPTLKTPE
SVTGTWKGDVKIQCIYDPLRGYRQVLVKWLVRHGSDSVTIFLRDSTGDHIQ
QAKYRGRLKVSHKVPGDVSLQINTLQMDDRNHYTCEVTWQTPDGNQVIRD
KIIELRVRKYNPPRINTEAPTTLHSSLEATTIMSSTSDLTTNGTGKLEETIAGSG
RNLPG

[SEQ ID NO: 10] VEGF binder-mu IgG2a Fc DANG-mu CRIg

SDTGRPFVEMYSEIPEIIHMTEGRELVIPCRVTSPNITVTLKKFP

LDTLIPDGKRIIWDSRKGFIISNATYKEIGLLTCEATVNGHLYKTNYLTHRQT

NTIIDVVLSPSHGIELSVGEKLVLNCTARTELNVGIDFNWEYPSSKHQHKKLV

NRDLKTQSGSEMKKFLSTLTIDGVTRSDQGLYTCAASSGLMTKKNSTFVRV

HEKGGGGSEPRGPTIKPCPPCKCPAPNLLGGPSVFIFPPKIKDVLMISLSPIVTC

VVVAVSEDDPDVQISWFVNNVEVHTAQTQTHREDYGSTLRVVSALPIQHQD

WMSGKEFKCKVNNKDLPAPIERTISKPKGSVRAPQVYVLPPPEEEMTKKQVT

LTCMVTDFMPEDIYVEWTNNGKTELNYKNTEPVLDSDGSYFMYSKLRVEK

KNWVERNSYSCSVVHEGLHNHHTTKSFSRTPGGGGGSGGGGSHPTLKTPES

VTGTWKGDVKIQCIYDPLRGYRQVLVKWLVRHGSDSVTIFLRDSTGDHIQQ

AKYRGRLKVSHKVPGDVSLQINTLQMDDRNHYTCEVTWQTPDGNQVIRDKI

IELRVRKYNPPRINTEAPTTLHSSLEATTIMSSTSDLTTNGTGKLEETIAGSGR

NLPG

**[SEQ ID NO: 11] mu IgG2a Fc DANG**

APNLLGGPSVFIFPPKIKDVLMISLSPIVTCVVVAVSEDDPDVQI

SWFVNNVEVHTAQTQTHREDYGSTLRVVSALPIQHQDWMSGKEFKCKVNN

KDLPAPIERTISKPKGSVRAPQVYVLPPPEEEMTKKQVTLTCMVTDFMPEDIY

VEWTNNGKTELNYKNTEPVLDSDGSYFMYSKLRVEKKNWVERNSYSCSVV

HEGLHNHHTTKSFSRTPGK

**[SEQ ID NO: 12] mu IgG2a Fc DANG-VEGF binder**

EPRGPTIKPCPPCKCPAPNLLGGPSVFIFPPKIKDVLMISLSPIVT

CVVVAVSEDDPDVQISWFVNNVEVHTAQTQTHREDYGSTLRVVSALPIQHQ

DWMSGKEFKCKVNNKDLPAPIERTISKPKGSVRAPQVYVLPPPEEEMTKKQ

VTLTCMVTDFMPEDIYVEWTNNGKTELNYKNTEPVLDSDGSYFMYSKLRVE

KKNWVERNSYSCSVVHEGLHNHHTTKSFSRTPGGGGGSGGGGSSDTGRPFV

EMYSEIPEIIHMTEGRELVIPCRVTSPNITVTLKKFPLDTLIPDGKRIIWDSRKG

FIISNATYKEIGLLTCEATVNGHLYKTNYLTHRQTNTIIDVVLSPSHGIELSVG

EKLVLNCTARTELNVGIDFNWEYPSSKHQHKKLVNRDLKTQSGSEMKKFLS

TLTIDGVTRSDQGLYTCAASSGLMTKKNSTFVRVHEK

[SEQ ID NO: 13] Aflibercept

SDTGRPFVEMYSEIPEIIHMTEGRELVIPCRVTSPNITVTLKKFP

LDTLIPDGKRIIWDSRKGFIISNATYKEIGLLTCEATVNGHLYKTNYLTHRQT

NTIIDVVLSPSHGIELSVGEKLVLNCTARTELNVGIDFNWEYPSSKHQHKKLV

NRDLKTQSGSEMKKFLSTLTIDGVTRSDQGLYTCAASSGLMTKKNSTFVRV

HEKDKTHTCPPCPAPELLGGPSVFLFPPKPKDTLMISRTPEVTCVVVDVSHED

PEVKFNWYVDGVEVHNAKTKPREEQYNSTYRVVSVLTVLHQDWLNGKEY

KCKVSNKALPAPIEKTISKAKGQPREPQVYTLPPSRDELTKNQVSLTCLVKGF

YPSDIAVEWESNGQPENNYKTTPPVLDSDGSFFLYSKLTVDKSRWQQGNVFS

CSVMHEALHNHYTQKSLSLSPG

**Preparation Example 2. Preparation of human proteins (C1.01 to C1.06 and aflibercept) and mouse proteins (C1.03m and C1.04m)**

**Preparation Example 2.1. Vector construction and plasmid maxi-prep**

Tables 2 and 3 list the used reagents and equipment, as below.

[Table 2]

| Reagent | Manufacturer | Catalog # |
|---|---|---|
| pTT5 | chempartner | |
| In-Fusion HD cloning kit | Clontech | 639648 |
| Accuprime pfx DNA polymerase | Invitrogen | 12344-04 |
| Gel DNA fragment purication Kit | TaKaRa | D823A |
| FASTDIGEST® BamHI | Fermentas | FD0055 |
| FASTDIGEST® EcoRI | Fermentas | FD0275 |

[Table 3]

| Equipment and instrument | Manufacturer | Model name |
|---|---|---|
| Biosafety cabinet | NUAIRE | LabGard class II |
| Centrifuge | Eppendorf | 5424 |
| Gel imaging system | Tanon | 2500R |

The synthesized DNA fragment was amplified through PCR, and the PCR product was purified by gel. The pTT5 vector was cut with restriction enzymes EcoRI and BamHI, and then purified by gel. Each PCR product and the linear vector were ligated using the In-Fusion Kit. The produced vector was transformed in ECOS101 DH5α competent cells, and cultured on a 2xYT agar plate containing 100 μg/ml of ampicillin. All manipulation processes were performed according to standard transformation protocols. Positive recombinants were confirmed through colony PCR, and sequence-verify sequencing was performed on a recombinant plasmid. A single colony was selected and the spawn was inoculated into 5 mL of a 2xYT medium containing 100 μg/ml of ampicillin. Culturing was performed at 37°C for 8 hours with shaking.

Thereafter, the spawn was diluted in 200 mL of a selective 2xYT medium in a ratio of 1: 1,000. Culturing was performed at 37°C for 16 hours with shaking. Bacterial cells were collected through centrifugation at 4°C and 4,700 rpm for 10 minutes. The bacterial pellets were resuspended in 12 mL of a RES-EF buffer. Subsequently, 12 mL of an LYS-EF buffer was added, and the sealed tube was vigorously inverted to thoroughly mix, followed by culturing at room temperature for 5 minutes. 12 mL of a NEU-EF buffer was added to the lysate, and the tube was vigorously inverted to rapidly and thoroughly mix.

Before injecting the lysate into the NUCLEOBOND® Xtra column filter, a homogeneous suspension of precipitates was prepared by inverting the lysate tube three times, in order to prevent clogging of the filter. Subsequently, the NUCLEOBOND® Xtra column filter and the NUCLEOBOND® Xtra column were washed with 10 mL of a filter washing buffer FIL-EF. The NUCLEOBOND® Xtra column filter was taken out, or removed by inverting the column. The NUCLEOBOND® Xtra column was washed with 90 mL of a washing buffer ENDO.

The NUCLEOBOND® Xtra column was washed with 45 mL of a washing buffer WASH-EF. Plasmid DNA was eluted with 15 mL of an elution buffer ELU. The eluate was collected in a 50-mL centrifuge tube. 10.5 mL of room temperature isopropanol was added to precipitate the eluted plasmid DNA. After vortexing, the mixture was left for 2 minutes.

Thereafter, 5 mL of 70% ethanol was added to the pellets. Ethanol was carefully and completely removed from the tube using a pipette tip. The pellets were dried at room temperature (20°C to 25°C). Then, the DNA pellets were dissolved with 1,000 μl of $H_2O$.

**Preparation Example 2.2. Cell transfection and protein expression**

Table 4 lists the used materials and reagents, as below.

[Table 4]

| Material and reagent | Manufacturer (Product #) |
|---|---|
| 293F cells | Invitrogen (R790-07) |
| OPM 293 | OPM (81075-001) |
| PLURONIC® F-68, 10% (100X) | Gibco (24040-032) |
| 1 mg/ml PEI | Polyscience (23966) |
| OPTI MEM I | Gibco (31985088) |
| Peptone (20x) | FLUKA (P0521-1KG) |
| Shaker flask | |
| ISF1-X Incubator shaker | Kuhner shaker |

The 293F seed strain containing a complete medium was maintained in an incubator shaker at 130 rpm, 37°C, and 8% $CO_2$. Cells were cultured at a density of $0.3 \times 10^6$ cells/ml to $0.4 \times 10^6$ cells/ml, and the medium was replaced every 2 to 3 days. 24 hours before transfection, freshly subcultured 293F cells were prepared at $2.6 \times 10^6$ cells/ml. The prepared cells were cultured in an incubator shaker at 130 rpm, 37°C, and 8% $CO_2$. On the day of transfection, the density of the cells was adjusted to $5.0 \times 10^6$ cells/ml using a fresh medium. The adjustment was performed with a total volume of 1 L in a 3-L shaker flask. 0.4 mg of HC and 0.6 mg of an LC plasmid were diluted with 50 ml of OPTI MEM I, and filtered with a filter of 0.22 μm. Then, 2 mg of PEI was diluted with 50 ml of OPTI MEM I to prepare a transfection reagent.

The diluted PEI was added to the DNA mixture, and then immediately mixed. Subsequently, culturing was performed at room temperature for 15 minutes. The DNA-PEI mixture was added to the 293F cells prepared at $2.6 \times 10^6$ cells/ml. The cells were then continuously cultured for 24 hours in an incubator shaker at 130 rpm, 37°C, and 8%

$CO_2$. 24 hours after transfection, 10% peptone was added to 1/20 of the culture medium so that the final concentration was 0.5%. The cells were then continuously cultured in an incubator shaker at 130 rpm, 37°C, and 8% $CO_2$. The density/viability of the cells was daily measured and recorded, over a period of 2 to 5 days after the transfection. The cells were collected for purification 7 days after the transfection or when the cell viability was less than 70%.

**Preparation Example 2.3. Protein purification**

Tables 5 to 7 show the reagents, composition of each buffer, and equipment, which are used for protein purification, as below.

[Table 5]

| Reagent | Manufacturer | Catalog # |
|---|---|---|
| Mabselect SuRe | GE Healthcare | 11003493 |
| Tris | SIGMA | 77-86-1 |
| NaCl | ACROS ORGANIVS | 7647-14-5 |
| Sodium citrate | Adamas-beta | 76198B |
| Citric acid | GENERAL-Reagent | G83162B |
| Arginine | VETEC | V900343-500G |
| Succinic acid | Sigma-Aldrich | S9512-500G |
| Triton X-100 | ABCONE | X10010-1L |
| TRITON X-114 | SIGMA-ALDRICH | X114 |
| Millex-GP Filter Unit, 0.22 $\mu$m, Sterile | MILLIPORE | SLGP033RS |
| NaOH | Merck | B146369740 |

[Table 6]

| Buffer A | 25 mM Tris, 150 mM NaCl, pH 8.0 |
|---|---|
| Buffer B | 25 mM Tris, 150 mM NaCl, 0.1% Triton X-100, 0.1% Triton X-114, pH 8.0 |
| Buffer C | 100 mM Sodium Citrate, 150 mM NaCl, pH 3.0 |
| Buffer D | 1 M Arginine, 400 mM Succinic acid, pH 9.0 |
| Buffer E | 20 mM PB, pH 6.5, 1 M $(NH_4)_2SO_4$ |
| Buffer F | 20 mM PB, pH 6.5, 25% isopropyl alcohol |
| Final buffer | 20 mM HEPES, pH 7.5, 240 mM sucrose, or 20 mM His acetate pH 5.5, 240 mM sucrose |

[Table 7]

| Instruments | Manufacturer | Model name |
|---|---|---|
| AKTA Pure | GE Healthcare | 29-0182-24 |
| Centrifuge | Beckman | J-26xp |
| Gel imaging system | Tanon | 2500R |
| Sartopore 2 filter | Sartorius | 5445307H9-OO-A |

The proteins were purified using a Mabselect sure column. Specifically, the supernatant was collected through centrifugation at 4°C and 2,000 xg for 20 minutes. Subsequently, the supernatant was filtered with the Sartopore 2 filter. The clarified supernatant was loaded onto a 5-ml MabSelect Sure column equilibrated with the buffer A. The column was then washed with the buffer A until the A280 absorbance reached a baseline. The column was washed with 10 CV of the buffer B. The column was washed with 10 CV of the buffer A. The bound proteins were eluted with 6 CV of the buffer C, 1/6 volume of the buffer D was added to neutralize the eluents, and SDS-PAGE analysis and SEC-HPLC analysis were performed.

Thereafter, the proteins were purified through a HIC column. The proteins were then dialyzed against the buffer E at 4°C overnight. The supernatant was loaded onto a HIC column equilibrated with the buffer E. The column was then washed with the buffer E until the A280 absorbance reached a baseline. The bound proteins were eluted through gradient elution (10 CV of buffer F, 0% to 40%). The bound proteins were eluted with 2 CV of a 100% buffer F, and SDS-PAGE analysis was performed.

After purifying the proteins, the proteins were collected in one place, and then dialyzed against the final buffer at 4°C overnight. Subsequently, SDS-PAGE analysis and SEC-HPLC analysis were performed.

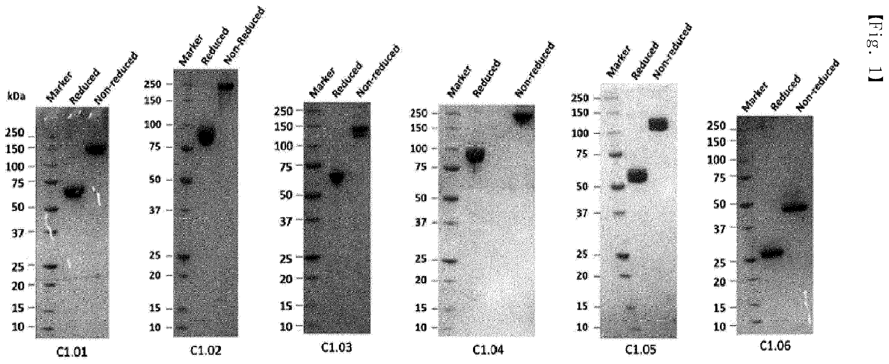

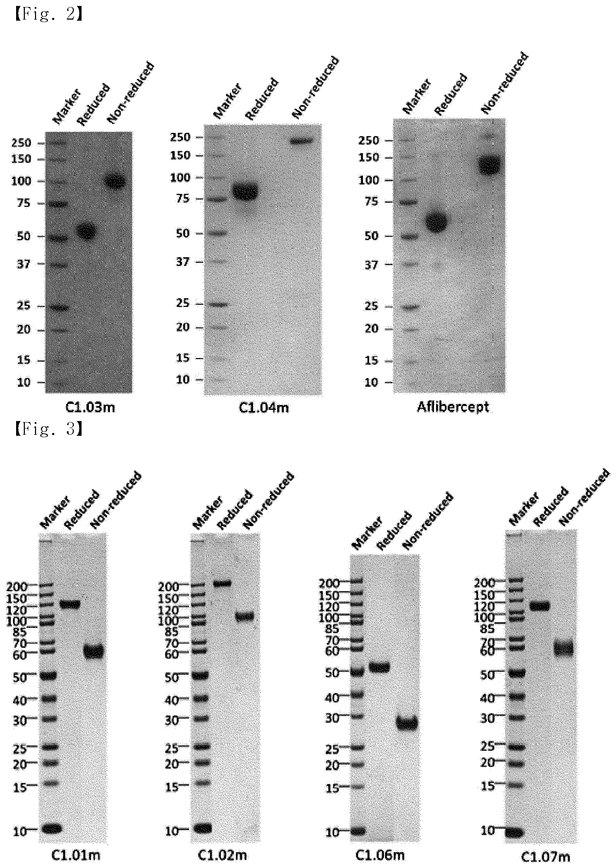

Consequently, as shown in FIGS. 1 and 2, the purified C1.01, C1.02, C1.03, C1.04, C1.05, C1.06, C1.03m, C1.04m, and aflibercept were confirmed through SDS-PAGE.

**Preparation Example 3. Preparation of mouse fusion proteins (C1.01m, C1.02m, C1.06m, and C1.07m)**

**Preparation Example 3.1. Preparation of protein**

Table 8 shows the used materials and reagents, as below.

[Table 8]

| Material and reagent | Vendor | Cat. # |
|---|---|---|
| EXPI293F™ Cells | Gibco | A14527 |
| EXPI293™ Expression System Kit | Gibco | A14635 |
| MabSelect SuRe | GE Lifesciences | 17543803 |
| Superdex 200 increase 10/300 | GE Lifesciences | 28990944 |
| MPC™ Ceramic Hydroxyfluoroapatite | Bio-rad | 1570200 |

A DNA fragment corresponding to the protein sequence was synthesized in Genewiz (No. 80-383034849). The corresponding DNA fragment was amplified through PCR, and introduced using a linearized pcDNA3.3 expression vector. The construction thereof was verified through sequencing, and then a sufficient amount of DNA to perform cell transfection was obtained through a large-scale plasmid preparation process.

First, Expi293F cells, of which 95% or more survived in a 2-L cell culture medium, were prepared at $2.94 \times 10^6$ cells/mL. Plasmid DNA and an EXPIFECTAMINE™ 293 reagent were first diluted in Opti-MEM, then mixed, and added to the cell culture medium. Cell culturing was performed in a platform shaker at a stirring speed of 150 rpm. The temperature was maintained at 37°C and the concentration of $CO_2$ was maintained at 8%. 18 to 20 hours after transfection, an enhancer 1 and an enhancer 2 were added to the cell culture medium.

After 6 days of the cell culturing, the cells were centrifuged at 4,000 rpm at 25°C for 10 minutes. The supernatant was collected for purification and gel electrophoresis. The supernatant was loaded onto SDS-PAGE gel according to the instructions for NUPAGE™ 4% to 12% Bis-Tris Protein Gels (ThermoFisher). A PAGERULER™ Unstained Protein Ladder (ThermoFisher) was used together with the protein samples in order to measure the molecular weights of the proteins. The remaining supernatant of each protein was used in the subsequent purification process.

The protein purification was performed as follows. Specifically, a protein A column was prepackaged together with a MabSelect Sure resin. Before loading the cell culture medium, the column was equilibrated with 0.1 M Tris (pH 7.0). After loading the cell culture medium, the column was washed with 0.1 M Tris (pH 7.0), and then eluted with 0.1 M glycine (pH 3.5). The eluents were neutralized by adding 0.1 M Tris (pH 9.0). Subsequently, the samples were dialyzed in PBS buffer (Sangon Biotech, B548117-0500).

Before loading the samples, an SEC column (GE lifesciences, Superdex 200 increase 10/300) was equilibrated with PBS. After the loading, the samples were eluted with PBS and collected through chromatography. SDS-PAGE was performed in order to analyze each peak thereof. Each sample was dialyzed in a formulation buffer (10 mM sodium phosphate, 0.3 to 0.4 M NaCl, pH 6.8).

A CHT column was prepackaged together with a CHT resin (Bio-rad, MPC™ Ceramic Hydroxyfluoroapatite), and equilibrated with the buffer A (10 mM sodium phosphate, 30 mM NaCl, pH 6.8) before loading the samples. After the loading, the column was eluted with a 30% buffer B (10 mM sodium phosphate, 1 M NaCl, pH 6.8), and then eluted with a 30% to 90% linear gradient buffer B and a final 100% of buffer B. The eluents were characterized by SDS-PAGE, and the samples were dialyzed in a formulation buffer (10 mM sodium phosphate, 0.3 to 0.4 M NaCl, pH 6.8). The final proteins were filtered with a filter of 0.2 $\mu$m, and aseptically dispensed in an amount of 0.5 mL into each of 1.5-mL tubes.

Consequently, as shown in FIG. 3, the purified C1.01m, C1.02m, C1.06m, and C1.07m were confirmed through SDS-PAGE.

### Preparation Example 3.2. Characterization of protein

The concentration of the protein was measured at 280 nm using a Nano Drop. The purity of the protein was confirmed through SDS-PAGE and HPLC-SEC.

An SDS-PAGE sample was prepared by mixing 15 $\mu$L of the purified protein and 5 $\mu$L of a 4x loading buffer, and boiling the mixture for 5 minutes. 15 $\mu$L of the mixed sample was loaded onto 4% to 12% gel of NuPAGE Bis-Tris Mini Gels. For the progress of SEC-HPLC analysis, 80 $\mu$L of the purified protein was loaded onto a TSKgel G3000SWxl column of HPLC system 1260 Infinity II, and 50 mM sodium phosphate (pH 7.0) and 150 mM sodium chloride were used as running buffers.

### Preparation Example 4. Purification of C3 and C3b of rabbit and rat

Preparation Example 4.1. Purification of C3 of rabbit and rat

Tables 9 to 11 show the reagents, composition of each buffer, and instrument, which are used for the purification of C3 of a rabbit and a rat, as below.

[Table 9]

| Material and reagent | Manufacturer | Cat. # |
|---|---|---|
| NaCl | ACROS ORGANIVS | 7647-14-5 |
| NaOH | Merck | B146369740 |
| $KH_2PO_4$ | GENERAL-Reagent | 7778-77-0 |
| $NaH_2PO_4$ | GENERAL-Reagent | 7558-80-7 |
| EDTA | GENERAL-Reagent | 60-00-4 |
| Benzamidine | Adamas | 618-39-3 |
| PBS | Gibco | C10010500BT |
| $MgCl_2$ | GENERAL-Reagent | 7786-30-3 |
| Factor B | Complement Technology | A135 |
| Factor D | Complement Technology | A136 |

[Table 10]

| DEAE column | |
|---|---|
| Buffer A | 10 mM Potassium phosphate ($KH_2PO_4$), 5 mM ethylenediaminetetraacetic acid disodium salt (EDTA), 1 mM benzamidine, pH 7.8 |
| Buffer B | 10 mM Potassium phosphate ($KH_2PO_4$), 5 mM EDTA, 1 mM benzamidine, 1 M sodium chloride (NaCl), pH 7.8 |
| MonoS column | |
| Buffer A | 50 mM Sodium phosphate ($NaH_2PO_4$) |
| Buffer B | 50 mM Sodium phosphate ($NaH_2PO_4$), 1 M sodium chloride (NaCl), pH 5.5 |
| S200 column | |
| Buffer | Phosphate-buffered saline (PBS), pH 7.4 |

[Table 11]

| Instruments | Manufacturer | Model name |
|---|---|---|
| AKTA Pure | GE Healthcare | 29-0182-24 |
| Centrifuge | Beckman | J-26xp |
| Gel imaging system | Tanon | 2500R |
| Millex-GP Filter Unit, 0.22 $\mu$m, Sterile | MILLIPORE | SLGP033RS |
| DEAE 16/10 FF | GE Healthcare | 10294207 |
| Mono S 5/50 GL | GE Healthcare | 10298955 |
| Sephadex S200 | GE Healthcare | 17104-302 |
| HPLC | Waters | E2695 |

Prior to protein purification, a step for removing high-molecular-weight substances from plasma was performed. The volume of the plasma was measured, and then 10% (ratio of weight/volume) $Na_2SO_4$ (anhydrous) of the powdered salt crystals was slowly added to the protein solution while stirring. Subsequently, stirring was performed at 4°C for 2 hours. After centrifugation was performed at 4°C and 26,892 xg for 30 minutes using a Sorvall Ultracentrifuge, the supernatant was obtained and the volume thereof was measured. The obtained supernatant was dialyzed against 5 L of a DEAE buffer A, and after 2 hours, replacement with 5 L of a new solution was performed. For the best results, the dialysis was performed at 4°C overnight. In order to confirm whether the dialysis was completed, the volume, A280, and conductivity of the dialysate were measured. When the solution was turbid or precipitated, centrifugation was performed at 4°C and 4,000 xg for 15 minutes to remove the precipitates. The dialysate was filtered with a filter of 0.2 $\mu$m, and stored on ice until purification progressed.

The protein purification was performed in the order of anion exchange chromatography, cation exchange chromatography, and size exclusion chromatography. Before loading the dialysate, a DEAE column (GE healthcare, HIPREP™ DEAE Fast Flow 16/10) was equilibrated with the buffer A (10 mM $KH_2PO_4$, 5 mM EDTA, 1 mM benzamidine, pH 7.8). After the loading, the column was washed with the buffer A, and eluted with a 0% to 50% linear gradient buffer B (10 mM $KH_2PO_4$, 5 mM EDTA, 1 mM benzamidine, 1 M NaCl, pH 7.8). The fractions of the eluent were analyzed through SDS-PAGE, the fractions containing C3 were collected, and the A280 thereof was measured. The obtained eluted substance was dialyzed in 2 L of a Mono S buffer A (50 mM sodium phosphate) at 4°C for 2 hours.

A Mono S column (GE healthcare, MONO S® 5/50 GL) was equilibrated with the buffer A (50 mM sodium phosphate) before loading the dialysate. After loading a DEAE eluate, the column was washed with the buffer A, and eluted with a 0% to 35% linear gradient buffer B (50 mM sodium phosphate, 1 M NaCl, pH 5.5). The fractions of the eluted substance were analyzed through SDS-PAGE, the centrally located peak fractions were pooled, and the A280 thereof was measured.

Before loading the samples, an SEC column (GE lifesciences, Superdex 200 increase 10/300) was equilibrated with PBS. After the loading, the samples were eluted with PBS and collected through chromatography. SDS-PAGE was performed in order to analyze each peak thereof. The centrally located peak C3 fractions were pooled, and the A280 thereof was measured.

Preparation Example 4.2. Purification of C3b of rabbit and rat

C3 was prepared by diluting with 0.5 mg/mL of PBS. The C3 was converted into C3b by adding a 0.4 $\mu$M factor B, a 0.05 $\mu$M factor D, and 5 mM $MgCl_2$ and culturing the resultant at 25°C for 30 minutes. The C3b was further purified through a Superdex 200 (60 mL) gel filtration column. Subsequently, SDS-PAGE analysis and SEC-HPLC analysis of rabbit C3, rabbit C3b, rat C3, and rat C3b were performed.

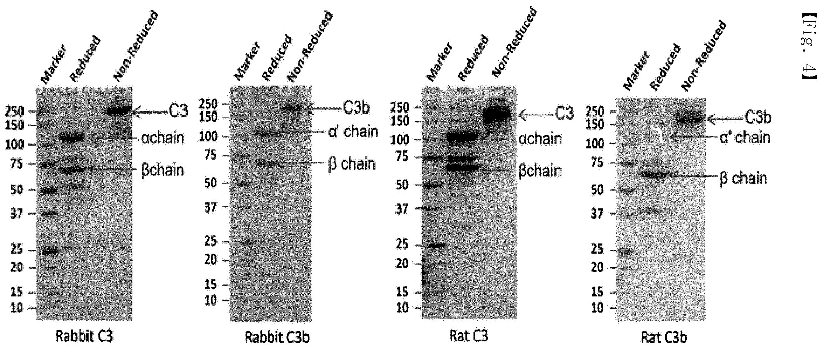

Consequently, as shown in FIG. 4, it was confirmed that the rabbit C3, the rabbit C3b, the rat C3, and the rat C3b were purified.

**Experimental Example 1. Measurement of binding force of fusion protein**

**Experimental Example 1.1. Biacore surface plasmon resonance (SPR) analysis technique**

In order to measure the binding force of the fusion protein, the physical properties of the prepared fusion protein were analyzed using Biacore 8K (GE Healthcare, 29129951) instrument.

Table 12 shows the used materials and reagents, as below.

[Table 12]

| Reagent | Manufacturer | Cat. # |
|---|---|---|
| CM5 sensor chip | GE Healthcare | 29-1496-03 |
| HBS-EP+ buffer (10 γ) | GE Healthcare | BR-1006-69 |
| Amine coupling kit | GE Healthcare | BR-1006-33 |
| Human antibody Capture Kit | GE Healthcare | 29234600 |
| 10 mM Glycine 1.5 | GE Healthcare | BR-1003-54 |
| C3b, C2, and C4 | Complement Technology | A114, A112, and A105 |

**Experimental Example 1.2. Immobilization of anti-human immunoglobulin G (Fc) antibody on CM5 sensor chip**

1 L of a 1x HBS-EP+ buffer was prepared by mixing 100 mL of a 10x HBS-EP+ buffer and 900 mL of Milli-Q water. After mixing 50 mM NHS and 200 mM EDC in a ratio of 1:1 for 420 seconds, a CM5 chip was activated at a flow rate of 10 μL/min. In order to reach an immobilization level of about 10,000 RU, 25 μg/mL of anti-human immunoglobulin G (Fc) antibody (acetate having pH 5.0) was injected at a rate of 10 μL/ml for 400 seconds. The remaining activated ester groups were blocked by injecting 1 M ethanolamine (pH 8.5) at a rate of 10 μL/min for 420 seconds. In order to stabilize the baseline, the sensor chip was washed with 1x HBS-EP+ at a rate of 10 μL/min for 16 hours.

**Experimental Example 1.3. Measurement of binding kinetics**

In order to stabilize the baseline, a startup cycle consisting of a sample step and a regeneration step was performed three times. Sample step: a 1x HBS-EP+ buffer was injected into flow cells at a flow rate of 30 μL/min for 120 seconds, followed by a cleavage phase for 120 seconds and a stabilization phase for 30 seconds. Regeneration step: 10 mM glycine (pH 1.5) was injected into flow cells at a rate of 30 μL/min for 30 seconds, followed by a stabilization step for 30 seconds.

Thereafter, the measurement of binding kinetics was performed in the following manner.

A C3b stock solution was diluted to 50 nM using a 1x HBS-EP+ buffer. Human VEGF165 was diluted to 5 nM using a 1x HBS-EP+ buffer. The 50 nM and 5 nM solutions were then diluted to 0.78125 nM and 0.078125 nM. In the sample step, the diluted antigens were injected into the flow cells at a rate of 30 μL/min. Two 0 nM antigens (1x HBS-EP+ buffer) were used and removed from the reference signal. Binding for 180 seconds and separation for 400 seconds were performed. After the separation time, a stabilization step for 60 seconds was performed. In the regeneration step, 10 mM glycine (pH 1.5) was injected into the flow cells at a rate of 30 μL/min for 30 seconds, followed by a stabilization step for 60 seconds.

**Experimental Example 1.4. Analysis and results**

After subtracting the reference and 0 nM values from the sample values, binding kinetics were calculated using Biacore Insight Evaluation Software (Version 2.0.15.12933) and 1:1 binding model for curved fitting.

Table 13 shows the binding affinities of the test substances, as below.

[Table 13]

| Capture 1 Solution | Analyte 1 Solution | ka (1/Ms) | kd (1/s) | KD (M) |
|---|---|---|---|---|
| 0.5 μg/ml C1.01 | C3b | 3.47E+06 | 1.21E-02 | 3.47E-09 |
| 2 μg/ml C1.02 | C3b | 3.66E+06 | 1.59E-02 | 4.34E-09 |
| 2 μg/ml C1.02 | Human VEGF165 | 3.94E+06 | 2.47E-04 | 6.28E-11 |

The binding affinity of C1.01 and C1.02 to human C3b and human VEGF165 and the binding affinity of C1.01m and C1.02m to mouse C3b and human VEGF165 were measured through Biacore analysis.

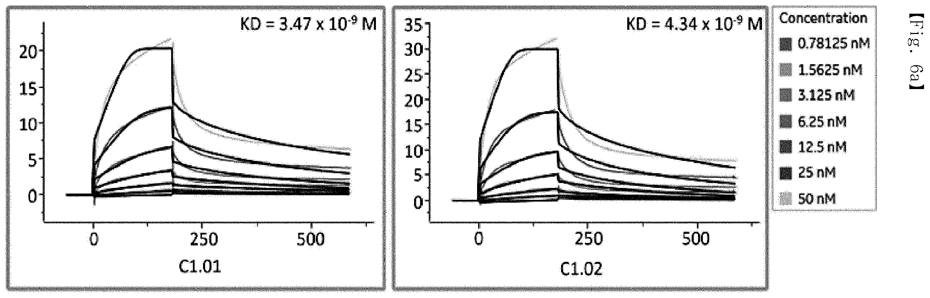

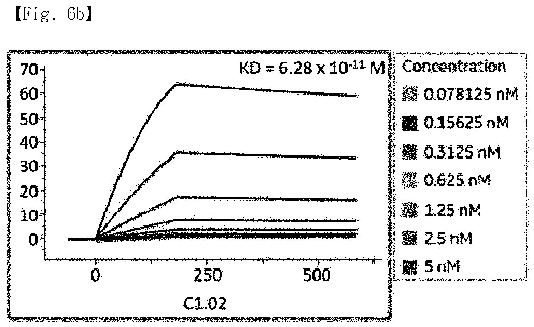

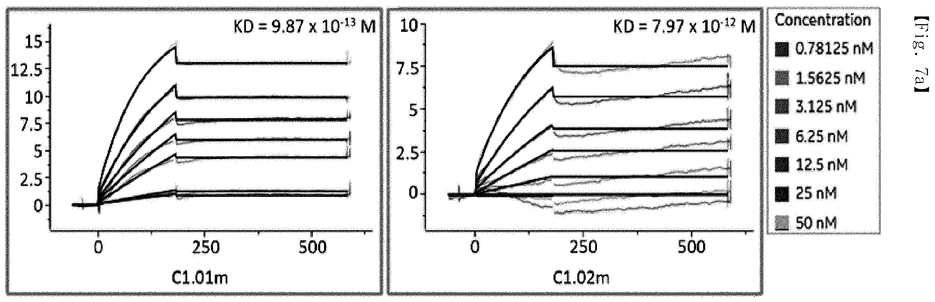

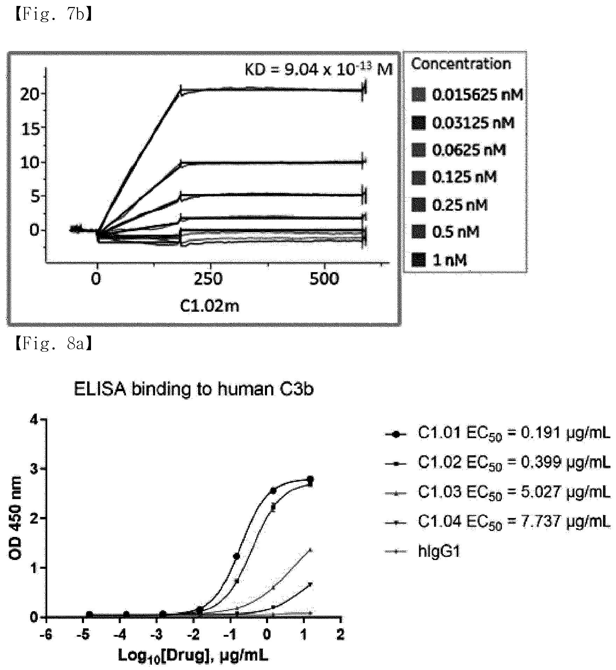

Consequently, as shown in Table 13 and FIGS. 6 and 7, it was confirmed that the C1.01 and C1.02 of the human and mouse both had a high binding affinity to the C3b, and the C1.02 of the human and mouse had a high binding affinity to the human VEGF165.

**Experimental Example 2. Measurement of binding affinity of fusion protein to complement protein and VEGF through enzyme-linked immunosorbent assay (ELISA)**

In order to confirm whether the complement pathway is inhibited by the fusion protein dimer according to one embodiment, whether C1.01, C1.02, C1.03, C1.04, and C1.01m to C1.03m were bound to C3b proteins was analyzed through enzyme-linked immunosorbent assay.

Specifically, a human C3b protein was immobilized on a plate, and C1.01, C1.02, C1.03, and C1.04, and hIgG1 as a control were bound thereto. Next, an anti-human immunoglobulin G antibody and an anti-horseradish peroxidase (HRP) antibody were sequentially bound thereto. Moreover, a mouse C3b protein was immobilized on a plate, and C1.01m to C1.03m containing mouse CRIg were bound to mouse C3b.

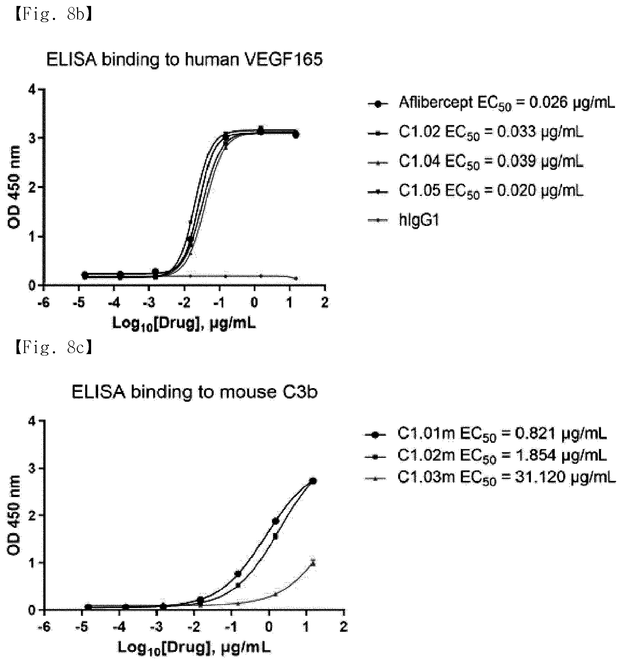

Consequently, as shown in FIG. 8a, it was confirmed that the C1.01, C1.02, C1.03, and C1.04 containing CRIg bound to the human C3b in a concentration dependent manner. Moreover, as shown in FIG. 8c, it was confirmed that the C1.01m to C1.03m containing mouse CRIg also bound to the mouse C3b in a concentration dependent manner.

In order to confirm whether the fusion protein according to one embodiment has an anti-VEGF action, whether the fusion protein and a human VEGF165 protein were bound was analyzed through enzyme-linked immunosorbent assay, and the binding affinity of aflibercept, C1.02, C1.04, and C1.05 to human VEGF 165 was measured through the ELISA.

Specifically, a human VEGF165 protein was immobilized on a plate, and C1.02, C1.04, and C1.05, and aflibercept and hIgG1 as controls were bound thereto. Next, an anti-human immunoglobulin G antibody and an anti-horseradish peroxidase (HRP) antibody were sequentially bound thereto.

Consequently, as shown in FIG. 8b, it was confirmed, as a result of measurement through the enzyme-linked immunosorbent assay, that the aflibercept, C1.02, C1.04, and C1.05 having anti-VEGF bound to the human VEGF165 in a concentration dependent manner.

In order to confirm that the fusion protein according to one embodiment binds to C3b of the alternative pathway to inhibit the alternative pathway, but does not bind to the human C2 or C4 protein of the classical pathway and thus does not inhibit the classical pathway, whether the fusion protein C1.01 according to one embodiment and the human Cb3, C2, and C4 proteins were bound was analyzed through ELISA.

Specifically, the human Cb3, C2, or C4 protein was immobilized on a plate, and C1.02 was bound thereto. Next, an anti-human immunoglobulin G antibody and an anti-horseradish peroxidase (HRP) antibody were sequentially bound thereto.

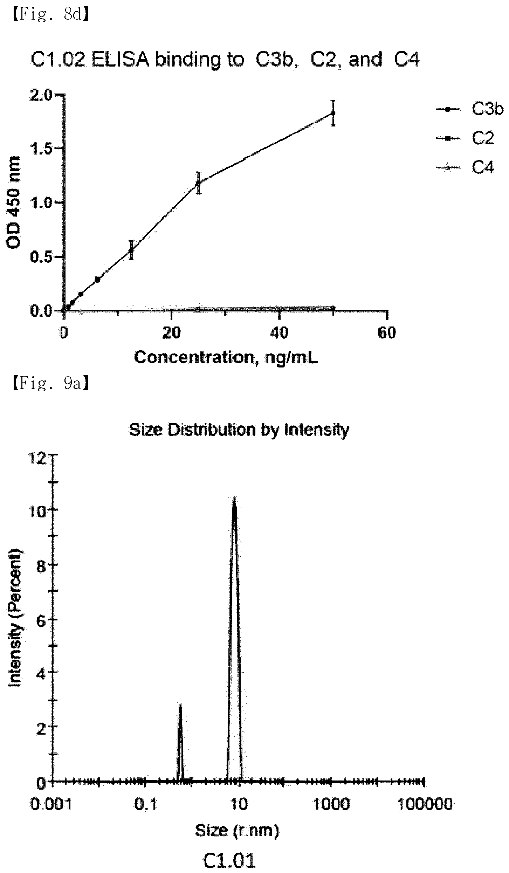

Consequently, as shown in FIG. 8d, it was confirmed that the C1.02 bound to the C3b, but did not bind to the C2 and C4.

**Experimental Example 3. Dynamic light scattering (DLS) analysis**

In order to measure the hydrodynamic radius of the fusion protein according to whether a VEGF binder is included, the hydrodynamic radii of C1.01 and C1.02 were analyzed through a dynamic light scattering method.

Specifically, C1.01 and C1.02 were centrifuged at 12,000 xg for 10 minutes, and the supernatant was added to a 96-well plate, followed by measurement at 25°C using Zetasizer APS (Marlvern) as DLS analysis equipment.

Table 14 is a table showing the hydrodynamic radii of C1.01 and C1.02, as below.

[Table 14]

|  |  | C1.01 | C1.02 |
|---|---|---|---|
| Z-Average (r.nm) | Test 1 | 5.828 | 8.603 |
|  | Test 2 | 6.058 | 8.686 |
|  | Test 3 | 6.078 | 8.596 |
|  | Mean | 5.988 | 8.628 |

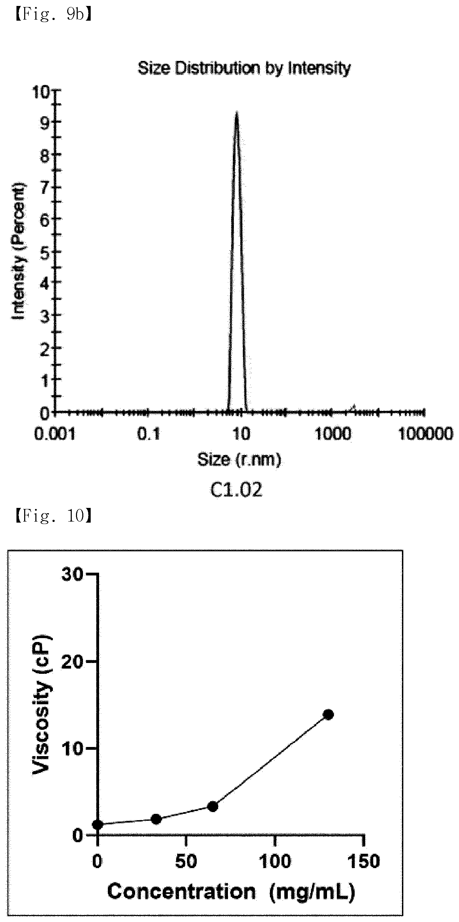

Consequently, as shown in Table 14 and FIGS. 9a and 9b, it was confirmed that the hydrodynamic radius of the C1.02 including the VEGF binder was measured longer than that of the C1.01 without VEGF binder.

**Experimental Example 4. Measurement of viscosity of C1.02**

In order to measure the viscosity of C1.02 by concentration, the viscosities of the C1.02 at 33, 65, and 130 mg/ml were measured using a micro-viscometer (m-VROC, RheoSence) and a vROC-mB05 (RheoSence) chip. Moreover, as a control, the viscosity of human serum albumin at 134 mg/ml was also measured.

Table 15 shows the measured viscosities, as below.

[Table 15]

| | Sample (concentration) | | | | |
|---|---|---|---|---|---|
| | C1.02 (0 mg/ml) | C1.02 (33 mg/ml) | C1.02 (65 mg/ml) | C1.02 (130 mg/ml) | Human serum albumin (134 mg/ml) |
| Viscosity (cP, mPa-s) | 1.235 | 1.851 | 3.327 | 13.912 | 2.194 |

Consequently, as shown in Table 15 and FIG. 10, it was confirmed that the viscosity of the C1.02 increased as the concentration thereof increased.

**Experimental Example 5. Analysis of alternative complement pathway inhibiting effect through hemolysis analysis**

Table 16 is a table showing the used reagents, as below.

[Table 16]

| Reagent | Manufacturer |
|---|---|
| C1q-depleted serum | Comptech |
| Rabbit RBC | Yuduo biolody |
| Gelatin Veronal Buffer (5 mM Barbital) | Boston bioproducts |
| Assay plate | Corning |

In order to confirm that the fusion protein according to one embodiment inhibits the alternative complement pathway, hemolysis analysis (AH50) of C1.01, C1.02, C1.04, C1.01m, C1.02m, and C1.04m was performed.

Specifically, 3 mL of rabbit red blood cells were washed with TBS using a centrifuge at 400 xg for 10 min, and after repeating this process twice, the rabbit red blood cells were washed once again with a GVB EGTA buffer using a centrifuge at 400 xg for 10 minutes. Next, the concentration of the rabbit red blood cells was adjusted to $1 \times 10^9$ cells/mL using a GVB EGTA buffer.

In order to analyze AH50 through the sensitized red blood cells, 9% human C1q-depleted serum was added to a 96-well plate (50 μL/well). Moreover, C1.01, C1.02, C1.01m, and C1.02m were treated at various concentrations. After culturing at 4°C for 30 minutes, rabbit red blood cells were added ($2 \times 10^6$ cells/well, 50 μL/well). Culturing was performed at 37°C for 1.5 hours, and centrifugation was performed at 600 xg for 10 minutes. After collecting 110 μL of the supernatant, the $OD_{415}$ value thereof was measured.

The alternative complement pathway inhibiting effects of C1.01, C1.02, C1.04, C1.01m, C1.02m, and C1.04m were confirmed through AH50 hemolysis analysis.

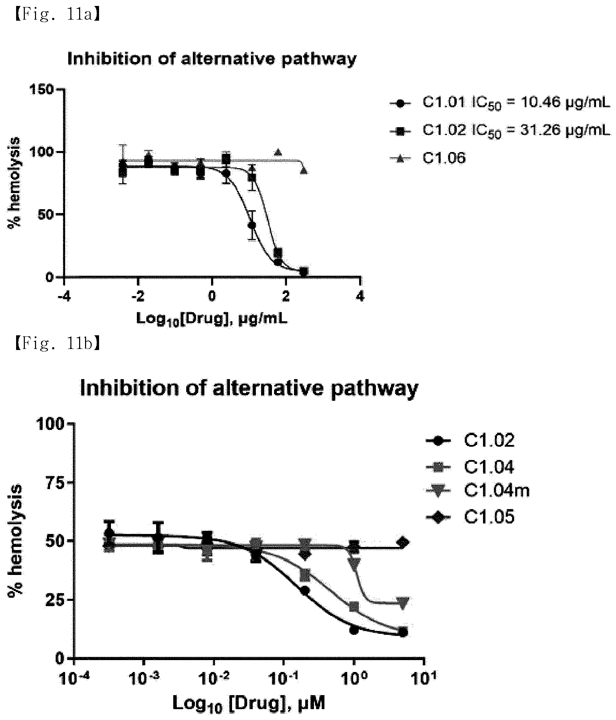

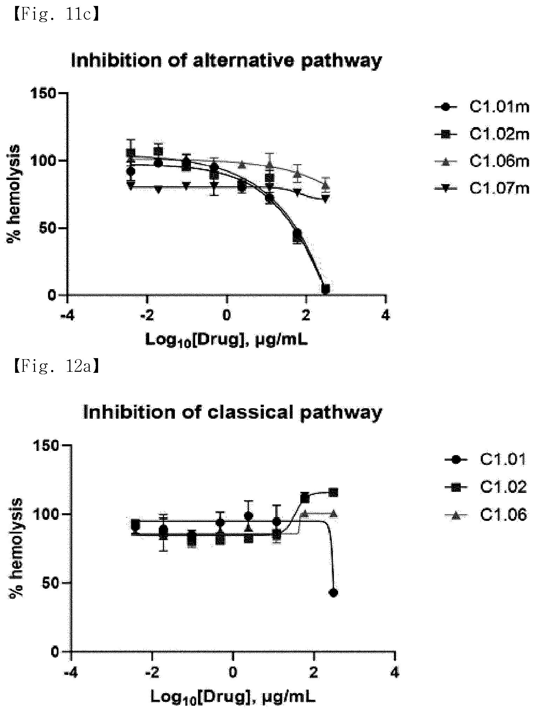

Consequently, as shown in Table 16 and FIGS. 11a to 11c, it was confirmed that the C1.01, C1.02, C1.04, C1.01m, C1.02m, and C1.04m inhibited the hemolytic action by the alternative complement pathway in a concentration dependent manner.

**Experimental Example 6. Analysis of classical complement pathway inhibiting effect through hemolysis analysis**

Table 17 is a table showing the used reagents, as below.

| Reagent | Manufacturer |
|---|---|
| Factor B-Dpl serum | Comptech |

(continued)

| Reagent | Manufacturer |
|---|---|
| Sheep RBC | Yuduo biolody |
| Hemolysin | Yuduo biolody |
| Gelatin Veronal Buffer (GVB; 5 mM Barbital) | Boston bioproducts |
| Assay plate | Corning |

In order to confirm that the fusion protein dimer according to one embodiment does not inhibit the classical complement pathway, hemolysis analysis (CH50) of C1.01, C1.02, C1.04, C1.05, C1.06, C1.01m, C1.02m, C1.04m, C1.06m, and C1.07m was performed.

Specifically, sheep red blood cells were centrifuged in TBS at 400 xg for 10 minutes, and after repeating this process twice, 1 mL of 20% sheep red blood cells, and hemolysins were incubated at 4°C for 30 minutes. Subsequently, the sheep red blood cells were washed with TBS using a centrifuge at 400 xg for 10 minutes, and after repeating this process twice, the sheep red blood cells were washed with a GVB++ buffer using a centrifuge at 400 xg for 10 minutes. Next, the concentration of the sensitized sheep red blood cells was adjusted to $1 \times 10^9$ cells/mL using a GVB++ buffer.

In order to analyze CH50 through the sensitized red blood cells, 3% or 4.5% human factor B-depleted serum was added to a 96-well plate (50 μL/well), and C1.01, C1.02, C1.06, C1.01m, C1.02m, C1.06m, and C1.07m were then treated at various concentrations. After culturing at 4°C for 30 minutes, the sensitized sheep red blood cells were added ($2.5 \times 10^6$ cells/well, 50 μL/well). Culturing was performed at 37°C for 30 minutes. Centrifugation was performed at 600 xg for 10 minutes, 110 μL of the supernatant was collected, and then the $OD_{415}$ value thereof was measured.

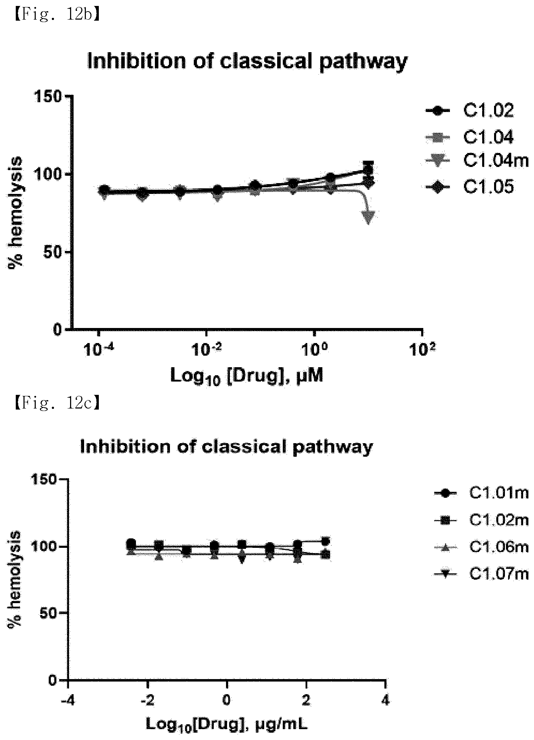

Consequently, as shown in Table 16 and FIGS. 12a to 12c, it was confirmed that the C1.01, C1.02, C1.04, C1.05, C1.06, C1.01m, C1.02m, C1.04m, C1.06m, and C1.07m all did not inhibit the hemolytic action by the classical complement pathway.

**Experimental Example 7. Efficacy analysis of fusion protein using VEGF reporter cells**

In order to confirm whether the fusion protein according to one embodiment effectively inhibits a VEGF protein, whether the binding to VEGF and a VEGF receptor was inhibited was analyzed.

The VEGF signaling inhibiting effects of aflibercept, C1.01, C1.02, C1.05, and C1.06 were confirmed using reporter cells. Specifically, it was analyzed, through the degree of luminescence, whether aflibercept, C1.02, and C1.05 inhibited the binding to VEGF and a VEGF receptor, using VEGF reporter cells (GA3001, Promega, USA) that generate luminescent light by receptor-mediated signaling when VEGF binds.

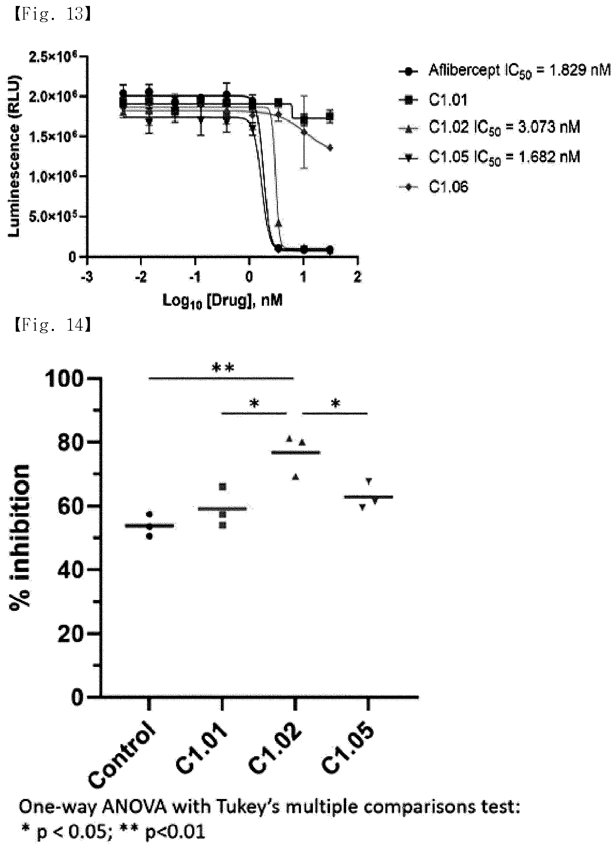

Consequently, as shown in FIG. 13, it was confirmed that the aflibercept, C1.02, and C1.05 inhibited the receptor-mediated signaling by the VEGF in a concentration dependent manner.

**Experimental Example 8. Evaluation of wound healing assay by fusion protein**

Whether the fusion protein dimer according to one embodiment effectively inhibits a VEGF protein was confirmed through the wound healing ability inhibiting effect of the fusion protein dimer.

Specifically, the VEGF signaling pathway inhibiting effects of C1.01, C1.02, and C1.05 were confirmed through a cell-based wound healing assay method. Adult retinal pigment epithelial cell line-19 (ARPE-19) was used for analysis. The ARPE-19 cells were cultured in Dulbecco's modified Eagle's medium (DMEM)/F12 medium using 10% fetal bovine serum (FBS). The ARPE-19 cells (80,000 cells/well) were cultured in a 24-well plate overnight. The next day, the respective wells were evenly wounded, VEGF (6 ng/ml) and C1.01, C1.02, and C1.05 (35 nM) were then added to the culture medium, and 24 hours later, the wound healing inhibiting ability of each fusion protein was analyzed.

Consequently, as shown in FIG. 14, it was confirmed that the C1.02 significantly inhibited wound healing compared to the control (p = 0.0031), and this effect was significant compared to the C1.01 (p = 0.0146) and the C1.05 (p = 0.0482).

**Experimental Example 9. Species cross reactivity test**

In order to confirm the species cross reactivity of the fusion protein dimer according to one embodiment, whether C1.02 bound to C3b and VEGFs derived from other species was analyzed through ELISA.

The binding ability of C3b of a human and a cynomolgus monkey to C1.02, the binding ability of C3b of a human and a cynomolgus monkey to C1.06, the binding ability of the VEGFs of a human, a cynomolgus monkey, a rat, and a rabbit to C1.02, and the binding ability of the VEGFs of a human, a cynomolgus monkey, a rat, and a rabbit to C1.01 were confirmed through ELISA. Specifically, the C3b or VEGF of a human, a cynomolgus monkey, a rat, or a rabbit was immobilized on a plate, and C1.01, C1.02, or C1.06 was bound thereto. Next, an anti-human immunoglobulin G antibody and an anti-horseradish peroxidase (HRP) antibody were sequentially bound thereto.

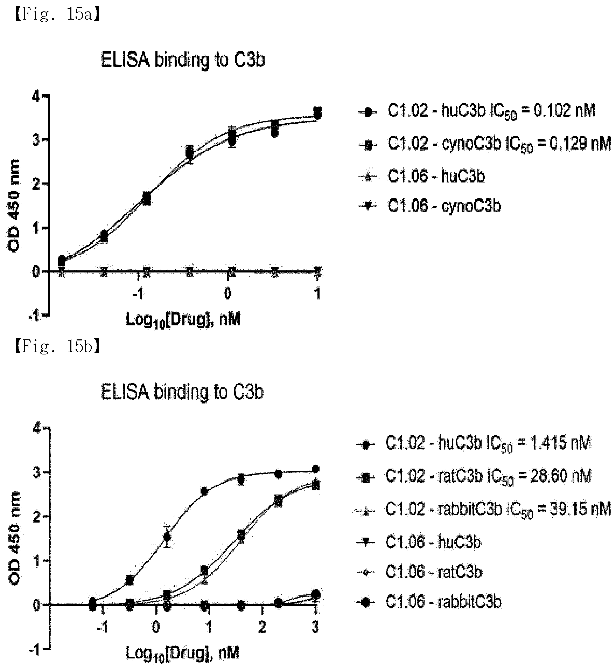

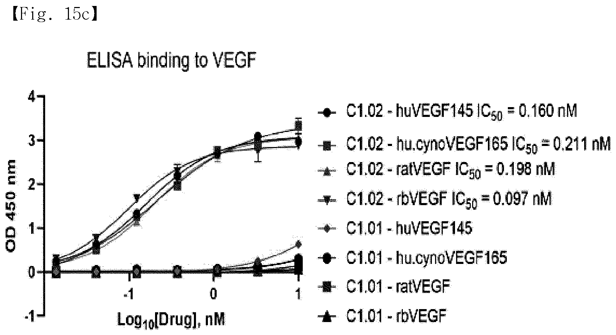

Consequently, as shown in FIGS. 15a to 15c, the C1.02 was confirmed to have species cross reactivity through the binding to the C3b and VEGFs of the cynomolgus monkey, rabbit, and rat.

**Experimental Example 10. Efficacy evaluation using mouse animal models for wet macular degeneration**

In order to confirm a therapeutic effect of the fusion protein according to one embodiment on wet macular degeneration (wet age-related macular degeneration), choroidal neovascularization was induced in mouse animal models, the fusion protein dimer according to one embodiment was then directly injected into the eyeball, and the effect was analyzed.

In order to induce the choroidal neovascularization phenotype in mice, whether there were structural abnormalities in the mice was confirmed through a spectral domain optical coherence tomography (Envisu R2200 SD-OCT System; Bioptigen, Inc., USA) before the induction.

Next, three choroidal neovascularization were induced by penetrating Bruch's membrane in the right eye of the mouse using a diode laser (OcuLight TX - Green 532 nm Laser; Iridex Corporate, USA) (Day 0). After the induction of the choroidal neovascularization, whether the models were successfully induced was confirmed using a spectral domain optical coherence tomography and fluorescein angiography (HRA2 FA system; Heidelberg Engineering GmbH, Germany).

Immediately after the induction, C1.02m (350 $\mu$M; 48.3 $\mu$g/$\mu$l, 2 $\mu$l), aflibercept (Eylea; 350 $\mu$M; 40.0 $\mu$g/$\mu$l, 2 $\mu$l),or a vehicle control (2 $\mu$l) was injected via an intravitreal injection. *In vivo* imaging was performed immediately after the induction (Day 0) of the choroidal neovascularization and 7 days after the induction (Day 7) using a spectral domain optical coherence tomography and a fluorescein angiography system. For each choroidal neovascularization in the photograph of the fluorescein angiography system, 1 point was scored if there was vascular leakage, and 0 points were scored if there was no vascular leakage, and then the lesion probability was calculated. For example, the notation of 100% means that vascular leakage was observed in the three choroidal neovascularization.

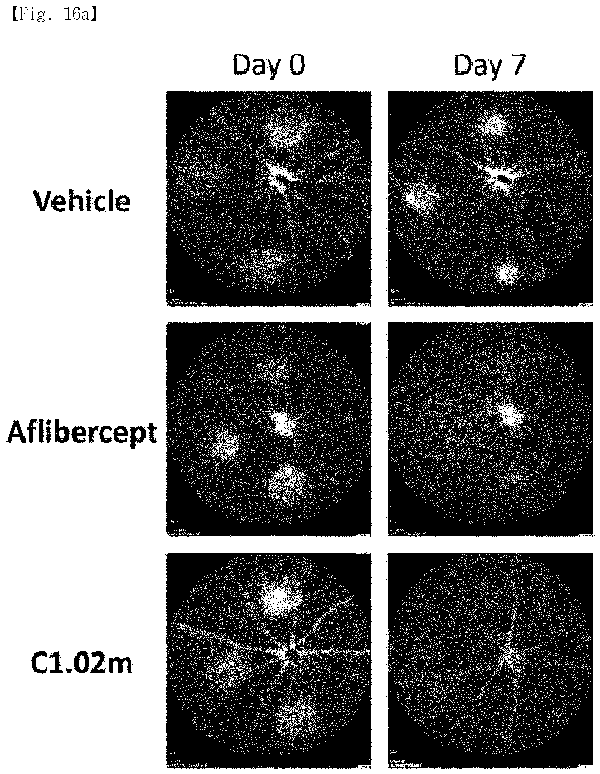

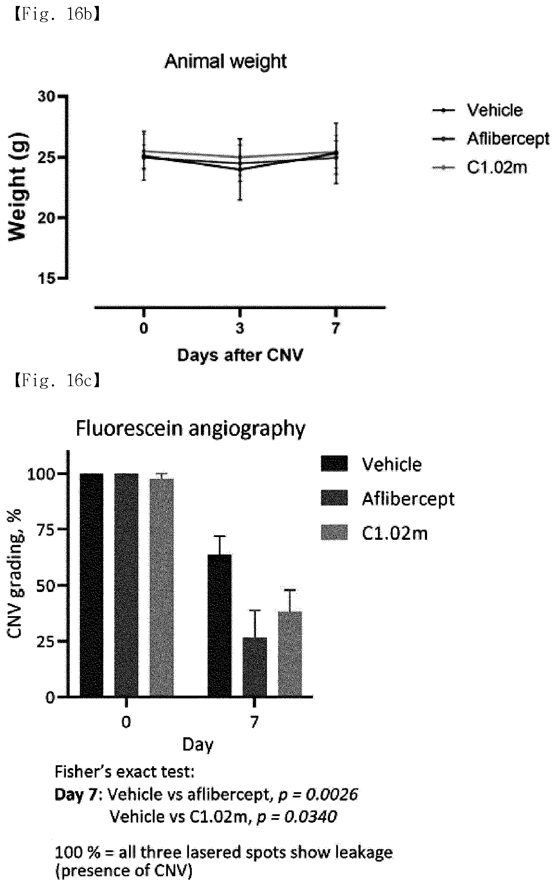

Consequently, as shown in FIGS. 16a to 16c, it was confirmed that after 7 days of the administration, the C1.02m group had a similar protective effect as the aflibercept group, and had a significant protective effect compared to the vehicle group.

**Experimental Example 11. Efficacy evaluation using rabbit animal models for wet macular degeneration**

In order to confirm a therapeutic effect of the fusion protein according to one embodiment on wet macular degeneration (wet age-related macular degeneration), choroidal neovascularization was induced in rabbit animal models, the fusion protein according to one embodiment was then directly injected into the eyeball, and the effect was analyzed.

Rabbit models for wet macular degeneration were prepared by inducing choroidal neovascularization in rabbits by a method similar to the aforementioned method in Experimental Example 10, except that 6 choroidal neovascularization were induced. Immediately after the induction of the choroidal neovascularization, C1.01 (350 $\mu$M; 38.45 $\mu$g/$\mu$l, 50 $\mu$l),aflibercept (350 $\mu$M; 40 $\mu$g/$\mu$l, 50 $\mu$l),or a vehicle control (50 $\mu$l) was injected via an intravitreal injection. *In vivo* imaging was performed immediately after the induction (Day 0) of the choroidal neovascularization, 7 days after the induction (Day 7), and 14 days after the induction (Day 14) using a fluorescein angiography system. The vascular leakage was measured by measuring fluorescein intensity in the choroidal neovascularization.

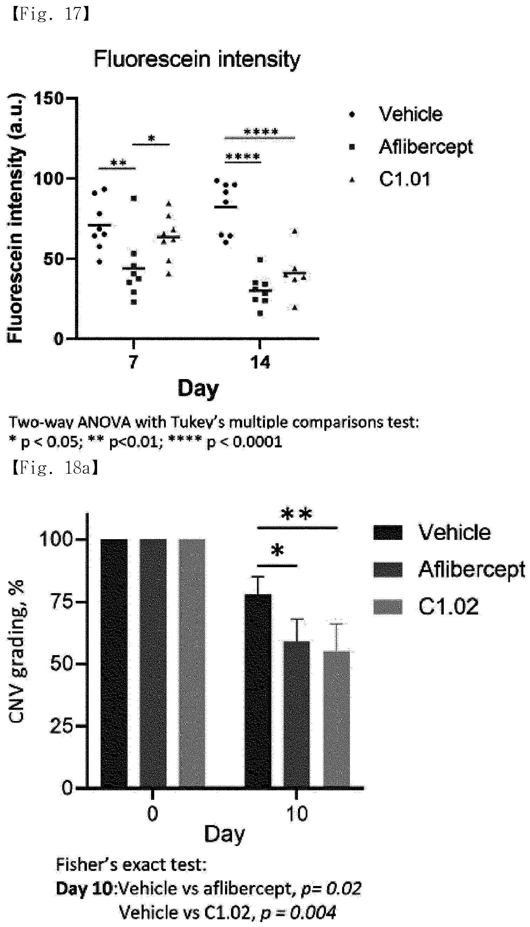

Consequently, as shown in FIG. 17, it was confirmed that after 7 days of the administration, the C1.01 group had a similar protective effect as the aflibercept group, and had a significant protective effect compared to the vehicle group.

**Experimental Example 12. Efficacy evaluation using rat animal models for wet macular degeneration**

In order to confirm a therapeutic effect of the fusion protein according to one embodiment on wet macular degeneration, choroidal neovascularization was induced in rat animal models, the fusion protein according to one embodiment was then directly injected into the eyeball, and the effect was analyzed.

Rat models for wet macular degeneration were prepared by inducing choroidal neovascularization in rats by a method similar to the aforementioned method in Experimental Example 10, except that 4 choroidal neovascularization were induced. Immediately after the induction of the choroidal neovascularization, C1.02 (350 $\mu$M; 54.93 $\mu$g/$\mu$l, 5 $\mu$l),aflibercept (350 $\mu$M; 40 $\mu$g/$\mu$l, 5 $\mu$l),or a vehicle control (5 $\mu$l) was injected via an intravitreal injection, and then *in vivo* imaging was performed on Day 0 and Day 10 using a fluorescein angiography system and a spectral domain optical coherence tomography. Through the imaging, the presence or absence of choroidal neovascularization and vascular leakage area immediately after the induction and 10 days after the induction were quantified and shown.

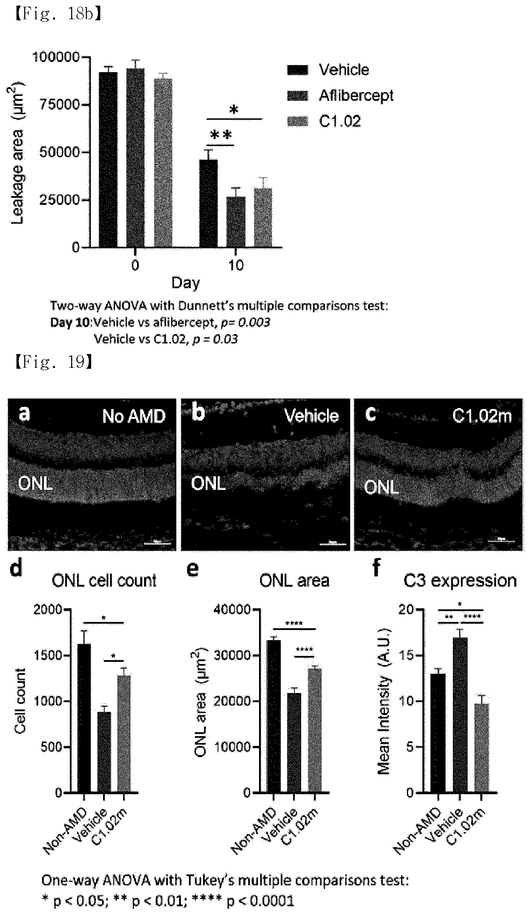

Consequently, as shown in FIGS. 18a and 18b, it was confirmed that after 10 days of the administration, the number of choroidal neovascularization and the vascular leakage area were significantly reduced in the C1.02 group so that the C1.02 group had a similar protective effect as the aflibercept group and had a significant protective effect compared to the vehicle group.

**Experimental Example 13. Efficacy evaluation using mouse animal models for dry macular degeneration**

In order to confirm a therapeutic effect of the fusion protein according to one embodiment on dry macular degeneration (dry age-related macular degeneration), dry macular degeneration was induced in mouse animal models, the fusion protein according to one embodiment was then directly injected into the eyeball, and the effect was analyzed.

First, mouse animal models for dry macular degeneration were induced by administering 20 mg/kg of sodium iodate ($NaIO_3$) to 8-week-old C57BL/6 mice via a tail vein injection.

After the model induction, C1.02m (260 $\mu$M; 36.1 $\mu$g/$\mu$l, 1.5 $\mu$l) or a vehicle control (1.5 $\mu$l) was administered on Day 0 and Day 7 via an intravitreal injection. 2 weeks after the model induction, the experimental animals were euthanized, and the eyes were then enucleated and fixed in Davidson's solution at 4°C for 24 hours. Subsequently, the fixed samples were stored in a 30% sucrose solution at 4°C for 3 days. The samples were frozen in OCT compound (Cat#4583, Sakura), and then sliced into a thickness of 20 $\mu$m. In the sliced tissues, a C3 (Cat #MA1-40046, Thermofisher) protein was stained using immunofluorescence and an outer nuclear layer (ONL) was stained using a DAPI (Cat #H-1200, Vector Laboratories) staining method. The stained samples were analyzed using a confocal microscope (LSM700; Zeiss, Germany). Specifically, an outer nuclear layer (ONL) of each experimental group of models for dry macular degeneration, the cell count in the outer nuclear layer, the area of the outer nuclear layer, and the C3 expression level in the retina were measured.

Consequently, as shown in FIGS. 19a to 19f, it was confirmed that, in the measurement of the cell count and area of the outer nuclear layer, retinal degeneration was significantly inhibited in the C1.02m group compared to the vehicle group. Moreover, it was confirmed that the C3 expression was significantly increased in the vehicle group compared to the non-AMD group, and the C3 expression was significantly decreased in the C1.02m group.

**Experimental Example 14. Analysis of pharmacokinetic profile of C1.02**

In order to analyze the pharmacokinetic profile of the fusion protein dimer according to one embodiment, 15 New Zealand white rabbits were grouped into five groups (G01 to G05) of three rabbits. After grouping, 2,500 $\mu$g of C1.02 (50 $\mu$l/eye) was administered via intravitreal injection.

In order to obtain samples for analyzing the pharmacokinetic profile, 0.5 ml of blood was first collected from the veins of rabbits at each hour. Plasma was separated from the obtained blood samples and then cryopreserved at -60°C. Moreover, vitreous humor (0.2 ml) and aqueous humor (0.2 ml) were obtained at each hour, and then cryopreserved at -60°C.

Table 18 shows the obtained rabbit samples by rabbit group, subject, and collection time, as below.

[Table 18]

| Group | Animal number | Dose route | Sample and tissue collection time points (Days) | | | | |
|---|---|---|---|---|---|---|---|
| | | | 1 hr | 24 hr | 3 days | 7 days | 14 days |
| G01 | RB 1001, RB 1002, RB1003 | Intravitreal injection | Vitreous humor, aqueous humor, plasma | | | | |
| G02 | RB2001, RB2002, RB2003 | | | Vitreous humor, aqueous humor, plasma | | | |
| G03 | RB3001, RB3002, | | | | Vitreous humor, | | |

(continued)

| Group | Animal number | Dose route | Sample and tissue collection time points (Days) | | | | |
|---|---|---|---|---|---|---|---|
| | | | 1 hr | 24 hr | 3 days | 7 days | 14 days |
| | RB3003 | | | | aqueous humor, plasma | | |
| G04 | RB4001, RB4002, RB4003 | | | | | Vitreous humor, aqueous humor, plasma | |
| G05 | RB5001, RB5002, RB5003 | | | | | | Vitreous humor, aqueous humor, plasma |

The concentrations of C1.02 in plasma, vitreous humor, and aqueous humor were measured using enzyme-linked immunosorbent assay (ELISA). Pharmacokinetic parameters were obtained by performing non-compartmental pharmacokinetic analysis with the measured values. Table 19 shows the pharmacokinetic parameters obtained by non-compartmental pharmacokinetic analysis, as below.

[Table 19]

| Matrix | Aqueous humor | Vitreous humor |
|---|---|---|
| PK parameters | Mean | Mean |
| $C_{max}$ (ng/mL) | 103705 | 3204272 |
| $T_{max}$ (h) | 1.00 | 1.00 |
| $T_{1/2}$ (h) | 114 | 226 |
| $T_{last}$ (h) | 168 | 336 |
| $AUC_{0-last}$ (ng.h/mL) | 10206790 | 545028800 |

Table 20 below is a table showing the concentration of C1.02 in the vitreous humor after administration of 2,500 µg of C1.02 to rabbits via intravitreal injection. RB# indicates an animal number, BQL indicates "below the quantifiable limit", and ND indicates "not determined".

[Table 20]

| | Time (h) | RBn001 * | RBn002 | RBn003 | Mean | SD | CV (%) |
|---|---|---|---|---|---|---|---|
| n = 1 | 1 | 2789070 | 3594376 | 3229369 | 3204272 | 403239 | 12.6 |
| n = 2 | 24 | 1561470 | 1336610 | 2160474 | 1686185 | 425856 | 25.3 |
| n = 3 | 72 | 1638252 | 2031203 | 2506814 | 2058756 | 434936 | 21.1 |
| n = 4 | 168 | 1659880 | 1767054 | 1783308 | 1736747 | 67064 | 3.86 |
| n = 5 | 1336 | 1206629 | 11286796 | 317153 | 936859 | 538176 | 57.4 |

Table 21 below is a table showing the concentration of C1.02 in the aqueous humor after administration of 2,500 µg of C1.02 to rabbits via an intravitreal injection. RB# indicates an animal number, BQL indicates "below the quantifiable limit", and ND indicates "not determined".

[Table 21]

| | Time (h) | RBn001* | RBn002 | RBn003 | Mean | SD | CV (%) |
|---|---|---|---|---|---|---|---|
| n = 1 | 1 | 1542 | 4803 | 304769 | 103705 | 174135 | 168 |
| n = 2 | 24 | 84982 | 54751 | 85448 | 75061 | 17590 | 23.4 |
| n = 3 | 72 | 38192 | 78324 | 90493 | 69003 | 27368 | 39.7 |
| n = 4 | 168 | 30505 | 37937 | 29392 | 32612 | 4646 | 14.2 |
| n = 5 | 336 | BQL** | BQL | BQL | ND*** | ND | ND |

Table 22 below is a table showing the concentration of C1.02 in the plasma after administration of 2,500 µg of C1.02 to rabbits via an intravitreal injection. RB# indicates an animal number, BQL indicates "below the quantifiable limit", and ND indicates "not determined".

[Table 22]

| | Time (h) | RBn001* | RBn002 | RBn003 | Mean | SD | CV (%) |
|---|---|---|---|---|---|---|---|
| n = 1 | 1 | BQL** | BQL | BQL | ND*** | ND | ND |
| n = 2 | 24 | BQL | BQL | BQL | ND | ND | ND |
| n = 3 | 72 | BQL | BQL | BQL | ND | ND | ND |
| n = 4 | 168 | BQL | BQL | BQL | ND | ND | ND |
| n = 5 | 336 | BQL | BQL | BQL | ND | ND | ND |

The concentrations of C1.02 in the vitreous humor and aqueous humor after administration of 2,500 µg of C1.02 to rabbits via an intravitreal injection were measured.

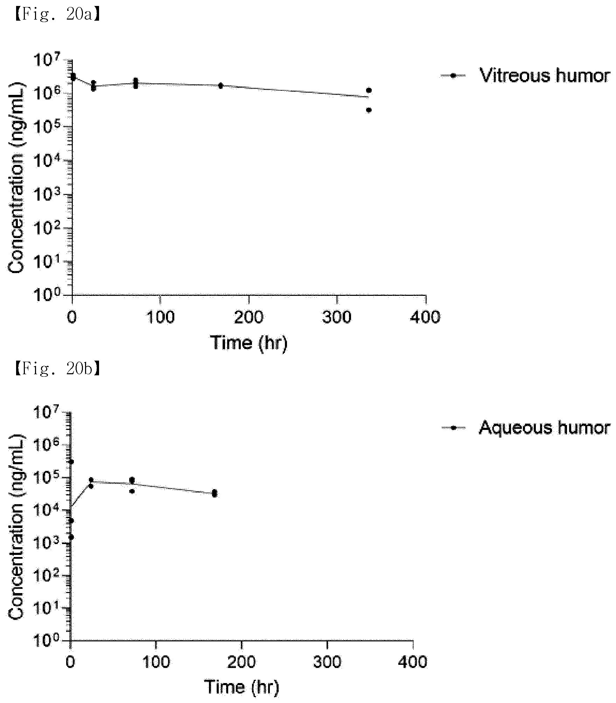

Consequently, as shown in Tables 20 to 22 and FIGS. 20a and 20b, it was confirmed that when 2,500 µg of C1.02 was administered via an intravitreal injection, the half-life in the vitreous humor was 226 hours and the half-life in the aqueous humor was 114 hours. Since no significant concentrations were observed in the plasma, the pharmacokinetic profile analysis was not possible in the plasma.

**Claims**

1. A fusion protein comprising:

    an extracellular domain of complement receptor of the immunoglobulin superfamily (CRIg) or a fragment thereof; and
    a protein that specifically binds to vascular endothelial growth factor (VEGF).

2. The fusion protein of claim 1, wherein the extracellular domain of the CRIg or a fragment thereof, and the protein that specifically binds to VEGF are linked by a linker.

3. The fusion protein of claim 2, wherein the linker is a peptide linker, an immunoglobulin fragment, or a combination thereof.

4. The fusion protein of claim 3, wherein the immunoglobulin fragment comprises a DANG variation or an NG variation.

5. The fusion protein of claim 3, wherein the immunoglobulin fragment comprises the amino acid sequence of SEQ ID NO: 6, SEQ ID NO: 11, or SEQ ID NO: 76.

6. The fusion protein of claim 1, wherein the protein that specifically binds to VEGF comprises an antibody, which specifically binds to VEGF, or a fragment thereof; and an extracellular domain of a VEGF receptor.

7. The fusion protein of claim 6, wherein the VEGF receptor is VEGF receptor 1 or VEGF receptor 2.

8. The fusion protein of claim 7, wherein an extracellular domain of the VEGF receptor comprises the amino acid sequence of SEQ ID NO: 14.

9. The fusion protein of claim 1, wherein the fusion protein consists of Structural Formula (I) or (II):

$$\text{N'-X-[linker (1)]n-Fc domain-[linker (2)]m-Y-C'} \quad \text{(I)}$$

$$\text{N'-Y-[linker (1)]n-Fc domain-[linker (2)]m-X-C'} \quad \text{(II)}$$

in Structural Formulae (I) and (II),

N' is the N-terminus of the fusion protein,
C' is the C-terminus of the fusion protein,
X is the extracellular domain of the CRIg or a fragment thereof,
Y is the protein that specifically binds to a VEGF,
the linker (1) and the linker (2) are peptide linkers, and
n and m are each independently 0 or 1.

10. The fusion protein of claim 9, wherein the linker (1) comprises the amino acid sequence of SEQ ID NO: 15 or SEQ ID NO: 17.

11. The fusion protein of claim 9, wherein the linker (2) comprises the amino acid sequence of SEQ ID NO: 16 or SEQ ID NO: 18.

12. The fusion protein of claim 9, wherein the fusion protein consists of Structural Formula (I).

13. The fusion protein of claim 1, wherein the fusion protein is any one selected from the group consisting of SEQ ID NO: 2, SEQ ID NO: 4, SEQ ID NO: 8, and SEQ ID NO: 10.

14. A fusion protein dimer in which the two fusion proteins of any one of claims 1 to 13 are bound.

15. The fusion protein dimer of claim 14, wherein the fusion protein dimer is a homodimer.

16. A polynucleotide encoding the fusion protein of any one of claims 1 to 13.

17. The polynucleotide of claim 16, the polynucleotide is any one selected from the group consisting of SEQ ID NO: 24, SEQ ID NO: 26, SEQ ID NO: 30, and SEQ ID NO: 32.

18. A vector comprising the polynucleotide of claim 16.

19. A transformed cell into which the vector of claim 18 is introduced.

20. A pharmaceutical composition for treating or preventing an eye disease, comprising, as an active ingredient, the fusion protein of claim 1 or the fusion protein dimer of claim 14.

21. The pharmaceutical composition for treating or preventing an eye disease of claim 20, wherein the eye disease is any one selected from the group consisting of age-related macular degeneration (AMD), geographic atrophy (GA), choroidal neovascularization (CNV), uveitis, diabetic and other ischemia-related retinopathy, diabetic macular edema, pathological myopia, a von Hippel-Lindau disease, ocular histoplasmosis, central retinal vein occlusion (CRVO), corneal angiogenesis, and retinal angiogenesis.

22. The pharmaceutical composition for treating or preventing an eye disease of claim 20, further comprising a pharmaceutically acceptable carrier.

23. Use of a fusion protein or a dimer thereof for treating an eye disease, wherein the fusion protein comprises an extracellular domain of CRIg or a fragment thereof, and a protein that specifically binds to VEGF.

24. Use of a fusion protein or a dimer thereof for the manufacture of a medicament for treatment or prevention of an eye disease, wherein the fusion protein comprises an extracellular domain of CRIg or a fragment thereof, and a protein that specifically binds to VEGF.

25. A method for treating and/or preventing an eye disease, comprising administering a fusion protein or a dimer thereof to a subject,
wherein the fusion protein contains an extracellular domain of CRIg or a fragment thereof, and a protein that specifically binds to VEGF.

【Fig. 1】

【Fig. 2】

C1.03m　　　　C1.04m　　　　Aflibercept

【Fig. 3】

C1.01m　　　　C1.02m　　　　C1.06m　　　　C1.07m

【Fig. 4】

【Fig. 5】

【Fig. 6a】

【Fig. 6b】

C1.02

KD = 6.28 x 10⁻¹¹ M

Concentration
- 0.078125 nM
- 0.15625 nM
- 0.3125 nM
- 0.625 nM
- 1.25 nM
- 2.5 nM
- 5 nM

【Fig. 7a】

【Fig. 7b】

KD = 9.04 x 10$^{-13}$ M

| Concentration |
| --- |
| 0.015625 nM |
| 0.03125 nM |
| 0.0625 nM |
| 0.125 nM |
| 0.25 nM |
| 0.5 nM |
| 1 nM |

C1.02m

【Fig. 8a】

ELISA binding to human C3b

C1.01 EC$_{50}$ = 0.191 µg/mL
C1.02 EC$_{50}$ = 0.399 µg/mL
C1.03 EC$_{50}$ = 5.027 µg/mL
C1.04 EC$_{50}$ = 7.737 µg/mL
hIgG1

【Fig. 8b】

## ELISA binding to human VEGF165

- Aflibercept $EC_{50}$ = 0.026 µg/mL
- C1.02 $EC_{50}$ = 0.033 µg/mL
- C1.04 $EC_{50}$ = 0.039 µg/mL
- C1.05 $EC_{50}$ = 0.020 µg/mL
- hIgG1

【Fig. 8c】

## ELISA binding to mouse C3b

- C1.01m $EC_{50}$ = 0.821 µg/mL
- C1.02m $EC_{50}$ = 1.854 µg/mL
- C1.03m $EC_{50}$ = 31.120 µg/mL

【Fig. 8d】

## C1.02 ELISA binding to C3b, C2, and C4

【Fig. 9a】

## Size Distribution by Intensity

C1.01

【Fig. 9b】

Size Distribution by Intensity

C1.02

【Fig. 10】

【Fig. 11a】

Inhibition of alternative pathway

【Fig. 11b】

Inhibition of alternative pathway

【Fig. 11c】

**Inhibition of alternative pathway**

【Fig. 12a】

**Inhibition of classical pathway**

【Fig. 12b】

**Inhibition of classical pathway**

【Fig. 12c】

**Inhibition of classical pathway**

【Fig. 13】

【Fig. 14】

One-way ANOVA with Tukey's multiple comparisons test:
* p < 0.05; ** p<0.01

【Fig. 15a】

ELISA binding to C3b

【Fig. 15b】

ELISA binding to C3b

【Fig. 15c】

ELISA binding to VEGF

C1.02 - huVEGF145 $IC_{50}$ = 0.160 nM

C1.02 - hu.cynoVEGF165 $IC_{50}$ = 0.211 nM

C1.02 - ratVEGF $IC_{50}$ = 0.198 nM

C1.02 - rbVEGF $IC_{50}$ = 0.097 nM

C1.01 - huVEGF145

C1.01 - hu.cynoVEGF165

C1.01 - ratVEGF

C1.01 - rbVEGF

【Fig. 16a】

Day 0　　　Day 7

Vehicle

Aflibercept

C1.02m

【Fig. 16b】

## Animal weight

**Days after CNV**

— Vehicle
— Aflibercept
— C1.02m

【Fig. 16c】

## Fluorescein angiography

**Day**

■ Vehicle
■ Aflibercept
■ C1.02m

Fisher's exact test:
**Day 7**: Vehicle vs aflibercept, $p = 0.0026$
Vehicle vs C1.02m, $p = 0.0340$

100 % = all three lasered spots show leakage
(presence of CNV)

【Fig. 17】

Two-way ANOVA with Tukey's multiple comparisons test:
* p < 0.05; ** p<0.01; **** p < 0.0001

【Fig. 18a】

Fisher's exact test:
**Day 10**:Vehicle vs aflibercept, *p= 0.02*
Vehicle vs C1.02, *p = 0.004*

【Fig. 18b】

Two-way ANOVA with Dunnett's multiple comparisons test:
**Day 10:**Vehicle vs aflibercept, *p= 0.003*
Vehicle vs C1.02, *p = 0.03*

【Fig. 19】

One-way ANOVA with Tukey's multiple comparisons test:
* p < 0.05; ** p < 0.01; **** p < 0.0001

【Fig. 20a】

【Fig. 20b】

## INTERNATIONAL SEARCH REPORT

International application No.

**PCT/KR2021/008681**

**A. CLASSIFICATION OF SUBJECT MATTER**

**C07K 14/705**(2006.01)i; **C07K 16/22**(2006.01)i; **A61K 38/00**(2006.01)i; **A61P 27/02**(2006.01)i

According to International Patent Classification (IPC) or to both national classification and IPC

**B. FIELDS SEARCHED**

Minimum documentation searched (classification system followed by classification symbols)

C07K 14/705(2006.01); A61K 47/61(2017.01); A61K 47/68(2017.01); C07K 14/47(2006.01); C07K 16/22(2006.01); C07K 16/32(2006.01); C12N 15/11(2006.01); C12N 9/12(2006.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: CRIg 세포외도메인(complement receptor of the immunoglobulin superfamily extracellular domain), VEGF 결합 단백질(vascular endothelial growth factor binding domain), Fc 도메인(Fc domain), 융합 단백질(fusion protein), 안질환(ocular disease), 황반 변성(macular degeneration)

**C. DOCUMENTS CONSIDERED TO BE RELEVANT**

| Category* | Citation of document, with indication, where appropriate, of the relevant passages | Relevant to claim No. |
|---|---|---|
| Y | US 2018-0312819 A1 (AP BIOSCIENCES, INC. et al.) 01 November 2018 (2018-11-01)<br>See claims 35-37; paragraphs [0011]-[0157]; figure 1B; and SEQ ID NOs: 8, 11 and 39. | 1-19,23,24 |
| Y | WO 2006-042329 A2 (GENENTECH, INC.) 20 April 2006 (2006-04-20)<br>See claims 1-4, 22, 28, 42 and 43; pages 14 and 58; and SEQ ID NO: 2. | 1-19,23,24 |
| A | KR 10-2013-0130732 A (GENENTECH, INC.) 02 December 2013 (2013-12-02)<br>See abstract; and claims 1-30. | 1-19,23,24 |
| A | KR 10-2019-0138636 A (GENENTECH, INC.) 13 December 2019 (2019-12-13)<br>See abstract; and claims 1-158. | 1-19,23,24 |
| A | US 2014-0294837 A1 (SAMSUNG ELECTRONICS CO., LTD.) 02 October 2014 (2014-10-02)<br>See abstract; and claims 1-22. | 1-19,23,24 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 October 2021** | **22 October 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2021/008681** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a.  ☑  forming part of the international application as filed:

            ☑   in the form of an Annex C/ST.25 text file.

            ☐   on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

            ☐   in the form of an Annex C/ST.25 text file (Rule 13*ter*.1(a)).

            ☐   on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

## INTERNATIONAL SEARCH REPORT

International application No.

**PCT/KR2021/008681**

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **25**
because they relate to subject matter not required to be searched by this Authority, namely:

Claim 25 pertain to a method for treatment of the human body by therapy (PCT Article 17(2)(a)(i) and PCT Rule 39.1(iv)).

2. ☑ Claims Nos.: **21,22**
because they relate to parts of the international application that do not comply with the prescribed requirements to such an extent that no meaningful international search can be carried out, specifically:

Claims 21 and 22 refer to claims 20 which violates the manner of referring to dependent claims (PCT Rule 6.4(a)), and thus are unclear.

3. ☑ Claims Nos.: **20**
because they are dependent claims and are not drafted in accordance with the second and third sentences of Rule 6.4(a).

Form PCT/ISA/210 (continuation of first sheet) (July 2019)

EP 4 180 453 A1

**INTERNATIONAL SEARCH REPORT**
Information on patent family members

International application No.

**PCT/KR2021/008681**

| Patent document cited in search report | | | Publication date (day/month/year) | Patent family member(s) | | | Publication date (day/month/year) |
|---|---|---|---|---|---|---|---|
| US | 2018-0312819 | A1 | 01 November 2018 | AU | 2012-318288 | A1 | 20 June 2013 |
| | | | | AU | 2012-318288 | B2 | 17 September 2015 |
| | | | | BR | 112014013205 | A2 | 27 October 2020 |
| | | | | CA | 2857168 | A1 | 06 June 2013 |
| | | | | CA | 2857168 | C | 27 October 2020 |
| | | | | CN | 104159926 | A | 19 November 2014 |
| | | | | CN | 104159926 | B | 01 February 2019 |
| | | | | CN | 110078831 | A | 02 August 2019 |
| | | | | DK | 2785744 | T3 | 27 November 2017 |
| | | | | EP | 2785744 | A1 | 08 October 2014 |
| | | | | ES | 2651521 | T3 | 26 January 2018 |
| | | | | JP | 2015-500811 | A | 08 January 2015 |
| | | | | JP | 2017-189167 | A | 19 October 2017 |
| | | | | JP | 6138815 | B2 | 31 May 2017 |
| | | | | US | 2015-0079084 | A1 | 19 March 2015 |
| | | | | US | 9988611 | B2 | 05 June 2018 |
| | | | | WO | 2013-082563 | A1 | 06 June 2013 |
| | | | | WO | 2013-082563 | A8 | 26 June 2014 |
| WO | 2006-042329 | A2 | 20 April 2006 | AT | 541931 | T | 15 February 2012 |
| | | | | AU | 2014-243951 | B2 | 06 December 2018 |
| | | | | BR | PI0908854 | A2 | 24 September 2019 |
| | | | | CA | 2906006 | C | 05 November 2019 |
| | | | | CL | 2009000082 | A1 | 02 March 2012 |
| | | | | CN | 109045307 | A | 21 December 2018 |
| | | | | EP | 3858387 | A1 | 04 August 2021 |
| | | | | ES | 2616362 | T3 | 12 June 2017 |
| | | | | HK | 1201539 | A1 | 04 September 2015 |
| | | | | IL | 255015 | A | 28 February 2018 |
| | | | | JP | 2017-079751 | A | 18 May 2017 |
| | | | | KR | 10-2012-0064120 | A | 18 June 2012 |
| | | | | US | 2017-0000897 | A1 | 05 January 2017 |
| | | | | WO | 2014-160307 | A1 | 02 October 2014 |
| KR | 10-2013-0130732 | A | 02 December 2013 | AR | 083677 | A1 | 13 March 2013 |
| | | | | AU | 2011-323487 | A1 | 10 May 2012 |
| | | | | AU | 2016-256692 | A1 | 24 November 2016 |
| | | | | BR | 112013010769 | A2 | 24 September 2019 |
| | | | | CA | 2816805 | A1 | 10 May 2012 |
| | | | | CN | 103201393 | A | 10 July 2013 |
| | | | | CN | 103201393 | B | 18 January 2019 |
| | | | | DK | 2635704 | T3 | 19 June 2017 |
| | | | | EP | 2635704 | A1 | 11 September 2013 |
| | | | | EP | 2635704 | B1 | 19 April 2017 |
| | | | | EP | 3170905 | A1 | 24 May 2017 |
| | | | | ES | 2628806 | T3 | 04 August 2017 |
| | | | | HU | E032938 | T2 | 28 November 2017 |
| | | | | IL | 226063 | A | 27 June 2013 |
| | | | | IL | 226063 | D0 | 27 June 2013 |
| | | | | JP | 2013-544512 | A | 19 December 2013 |
| | | | | JP | 2019-216732 | A | 26 December 2019 |
| | | | | MX | 2013004879 | A | 02 July 2013 |

### INTERNATIONAL SEARCH REPORT
Information on patent family members

International application No.

**PCT/KR2021/008681**

| Patent document cited in search report | | | Publication date (day/month/year) | Patent family member(s) | | | Publication date (day/month/year) |
|---|---|---|---|---|---|---|---|
| | | | | MX | 348025 | B | 24 May 2017 |
| | | | | NZ | 610067 | A | 29 May 2015 |
| | | | | PL | 2635704 | T3 | 29 September 2017 |
| | | | | RU | 2013125230 | A | 10 December 2014 |
| | | | | RU | 2593954 | C2 | 10 August 2016 |
| | | | | SG | 10201605904 | A | 29 September 2016 |
| | | | | SG | 190078 | A1 | 28 June 2013 |
| | | | | SI | 2635704 | T1 | 31 July 2017 |
| | | | | SI | EP2635704 | T1 | 31 July 2017 |
| | | | | TW | 201250003 | A | 16 December 2012 |
| | | | | US | 2012-0107315 | A1 | 03 May 2012 |
| | | | | US | 2018-163262 | A1 | 14 June 2018 |
| | | | | WO | 2012-061421 | A1 | 10 May 2012 |
| | | | | ZA | 201303001 | B | 29 October 2014 |
| | | | | ZA | 201405359 | B | 25 May 2016 |
| KR | 10-2019-0138636 | A | 13 December 2019 | AR | 111249 | A1 | 19 June 2019 |
| | | | | AU | 2018-237359 | A1 | 10 October 2019 |
| | | | | AU | 2018-237359 | A1 | 27 September 2018 |
| | | | | BR | 112019019706 | A2 | 28 April 2020 |
| | | | | CA | 3056248 | A1 | 27 September 2018 |
| | | | | CL | 2019002658 | A1 | 27 December 2019 |
| | | | | CN | 110612124 | A | 24 December 2019 |
| | | | | CO | 2019011582 | A2 | 31 October 2019 |
| | | | | CR | 20190481 | A | 06 January 2020 |
| | | | | EP | 3600442 | A1 | 05 February 2020 |
| | | | | IL | 269378 | D0 | 28 November 2019 |
| | | | | JP | 2020-514376 | A | 21 May 2020 |
| | | | | MX | 2019011141 | A | 05 November 2019 |
| | | | | PE | 20191758 | A1 | 12 December 2019 |
| | | | | PH | 12019502149 | A1 | 29 June 2020 |
| | | | | RU | 2019132064 | A | 22 April 2021 |
| | | | | RU | 2019132064 | A3 | 26 July 2021 |
| | | | | SG | 11201908650 | A | 30 October 2019 |
| | | | | TW | 201838649 | A | 01 November 2018 |
| | | | | US | 2020-0002411 | A1 | 02 January 2020 |
| | | | | WO | 2018-175752 | A1 | 27 September 2018 |
| US | 2014-0294837 | A1 | 02 October 2014 | EP | 2784092 | A2 | 01 October 2014 |
| | | | | EP | 2784092 | A3 | 10 December 2014 |
| | | | | EP | 2784092 | B1 | 27 December 2017 |
| | | | | JP | 2014-193864 | A | 09 October 2014 |
| | | | | JP | 2018-048192 | A | 29 March 2018 |
| | | | | JP | 6636684 | B2 | 29 January 2020 |
| | | | | KR | 10-2014-0119317 | A | 10 October 2014 |
| | | | | KR | 10-2049990 | B1 | 03 December 2019 |
| | | | | US | 9650443 | B2 | 16 May 2017 |

Form PCT/ISA/210 (patent family annex) (July 2019)

**EP 4 180 453 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007536964 A
- US 9527925 B2
- US 8268314 B2
- US 20190167790 A1